# EUROPÄISCHE PATENTSCHRIFT

(11) **EP 3 411 099 B1**
(45) Veröffentlichungstag und Bekanntmachung des Hinweises auf die Patenterteilung: **19.02.2020**
(21) Anmeldenummer: 16810372.9
(22) Anmeldetag: 14.12.2016
(51) Int. Cl.: A61M 5/24, A61M 5/50

(54) **INJEKTIONSVORRICHTUNG**
INJECTION DEVICE
DISPOSITIF D'INJECTION

(30) Priorität: 03.02.2016 DE 202016100531 U
(43) Veröffentlichungstag der Anmeldung: 12.12.2018
(73) Patentinhaber: H&B Electronic GmbH & Co. KG, 75392 Deckenpfronn (DE)
(72) Erfinder: MORLOK, Tobias, 71159 Mötzingen (DE); WEBER, Wilfried, 72296 Schopfloch (DE); PETRY, Dariusz, 71134 Aidlingen (DE); STAUSS, Wolfgang, 72414 Rangendingen (DE)
(74) Vertreter: Wacker, Jost Oliver
(86) Internationale Anmeldenummer: PCT/EP2016/080932
(87) Internationale Veröffentlichungsnummer: WO 2017/133818

(56) Entgegenhaltungen:
- EP-A1- 2 910 268
- WO-A1-2010/127449
- DE-U1- 7 808 708
- DE-U1- 7 808 708
- DE-U1- 20 311 996
- DE-U1- 20 311 996
- DE-U1-202010 000 846
- DE-U1-202010 000 846
- DE-U1-202014 004 561
- DE-U1-202014 004 561
- FR-A1- 2 767 479
- US-A1- 2016 354 557
- US-B1- 6 454 743
- US-B2- 9 180 258

## Beschreibung

Die Erfindung betrifft eine Injektionsvorrichtung nach dem Oberbegriff des Anspruchs 1. Diese weist ein Gehäuse auf, in dem eine Spritze aufgenommen werden kann, die eine Injektionsnadel, eine Aufnahmekammer, einen Kolben und einen Spritzenstößel aufweist. Ferner ist an der Injektionsvorrichtung eine Steuerungsanordnung zur gesteuerten Betätigung der Spritze während eines Anwendungsablaufes vorgesehen, der wenigstens einen Einstechhub, einen Injektionshub und einen Rückholhub umfasst. Dabei weist die Steuerungsanordnung ein Betätigungselement zur Beaufschlagung durch eine manuelle Antriebskraft, eine gegenüber dem Gehäuse verschiebbare Spritzenaufnahme, an der die Aufnahmekammer festgelegt werden kann, sowie eine gegenüber dem Gehäuse verschiebbare Stößelaufnahme auf, an der der Spritzenstößel festgelegt werden kann. Darüber hinaus ist an der Injektionsvorrichtung eine Übertragungseinrichtung vorgesehen, die ein Zahnradgetriebe zur Koppelung der Stößelaufnahme mit dem Betätigungselement aufweist. Mittels des Zahnradgetriebes kann dabei die Stößelaufnahme in Abhängigkeit von einer Relativbewegung des Betätigungselementes angetrieben werden.

Aus der DE 202014004561 U1 ist eine Injektionsvorrichtung zum vereinfachten Verabreichen eines Medikamentes aus einer Spritze bekannt. Hierbei ist die gefüllte Spritze unzugänglich innerhalb der Injektionsvorrichtung aufgenommen. Ferner ist ein Betätigungselement vorgesehen, dass von einer manuellen Kraft beaufschlagt werden kann. Zwischen diesen Betätigungselement und einem Gehäuse der Injektionsvorrichtung ist dabei ein Zahnradgetriebe angeordnet, über das in Abhängigkeit von einer Relativbewegung des Betätigungselementes gegenüber dem Gehäuse eine Stößelaufnahme angetrieben werden kann, um die Spritze auszupressen.

Derartige Injektionsvorrichtungen werden zusammen mit dem darin aufgenommenen Medikament vertrieben, so dass die Injektionsvorrichtung seitens des Medikamentenherstellers beziehungsweise -abfüllers zur Verfügung gestellt werden muss. Somit muss die Injektionsvorrichtung auch gemeinsam mit dem Medikament gelagert und transportiert werden. Je nach Art des verwendeten Medikamentes kann es dabei erforderlich sein, dieses zusammen mit der Injektionsvorrichtung während der Lagerung oder des Transportes zudem zu kühlen. Aufgrund der festen Aufnahme der Spritze in der Injektionsvorrichtung wird der hierbei erforderliche Kühlraum nicht allein durch die Spritzen mit dem darin aufgenommenen Medikament definiert, sondern durch die erheblich größeren Injektionsvorrichtungen. Infolgedessen ist der Kühlraum für Medikamente, die mit einer derartigen Injektionsvorrichtung verabreicht werden, relativ groß. Zudem besteht am Markt ein Bedürfnis danach, die Injektionsvorrichtungen auch unabhängig vom jeweiligen Medikament vermarkten zu können.

DE 203 11 996 U1 zeigt dabei eine Injektionsvorrichtung, in die eine Spritze mit dem zu verabreichenden Medikament eingesetzt werden kann, um einen gesteuerten Einstech-, Injektions- und Rückholhub durch eine einfach gerichtete Kraftbeaufschlagung vornehmen zu können. Die Aufbringung der Antriebskraft erfolgt hierbei durch manuelle Betätigung eines Betätigungselementes, über das eine Spritzenaufnahme und eine Stößelaufnahme verschoben werden.

Ferner sind aus DE 202010000846 U1, DE 69839165 T1 und US 9,180,258 B2 Injektionsvorrichtungen mit einem jeweils selbsttätigen Antrieb bekannt, die ein zweiteiliges Außengehäuse aufweisen. Das Außengehäuse kann dabei zwischen einer Offen- und einer Schließstellung verlagert werden, wobei eine Betätigung des Antriebs in der Offenstellung des Außengehäuses verhindert wird.

Die Aufgabe der Erfindung ist es, bei einer gattungsgemäßen Injektionsvorrichtung die genannten Nachteile zu vermeiden und dessen von den Medikamenten unabhängige Vermarktung zu ermöglichen sowie die Lagerung und den Transport des darin verabreichten Medikamentes zu vereinfachen beziehungsweise die hierbei entstehenden Kosten zu verringern.

Diese Aufgabe wird durch eine Injektionsvorrichtung mit den Merkmalen des Anspruchs 1 gelöst. Dabei weist das Gehäuse ein erstes äußeres Gehäuseteil und ein zweites äußeres Gehäuseteil auf, die aneinandergehalten sind. Dabei können die beiden äußeren Gehäuseteile zwischen einer Offenstellung, in der die Spritzenaufnahme zugänglich ist, und einer Schließstellung, in der die Spritzenaufnahme verschlossen ist, zueinander verlagert werden. Hierzu ist zwischen dem ersten äußeren Gehäuseteil und dem zweiten äußeren Gehäuseteil ein Schwenklager vorgesehen. Auf diese Weise kann die Verlagerbarkeit der beiden Gehäuseteile zwischen der Offenstellung und der Schließstellung mit besonders einfachen und kostengünstigen Mitteln zur Verfügung gestellt werden. Zudem können die Gehäuseteile auf diese Weise kostengünstiger hergestellt werden, wie beispielsweise durch einen einzelnen Formenguss und ermöglichen in der Offenstellung eine leichtere Montage der übrigen Bauteile. Hierbei ist es günstig, wenn das Schwenklager ein Filmscharnier aufweist beziehungsweise durch ein solches gebildet ist. Durch ein derartiges, beispielsweise als Schmetterlingsscharnier ausgeführtes Filmscharnier, kann sowohl das Schwenklager als auch eine zweifache Vorspannung desselben besonders kostengünstig hergestellt werden. Alternativ hierzu kann das Schwenklager verrastbare Scharniermittel aufweisen. Dies ermöglicht eine getrennte Herstellung und einfache Montage beider äußeren Gehäuseteile. Hierdurch ist es möglich, die Injektionsvorrichtung unabhängig von der darin aufzunehmenden Spritze beziehungsweise dem darin enthaltenen Medikament zu vermarkten, zu lagern und zu transportieren. Die Injektionsvorrichtung kann dabei beispielsweise unabhängig vom jeweiligen Medikament in der Apotheke erhältlich sein. Das heißt der Arzt könnte dem Patienten bei Bedarf die Injektionsvorrichtung separat verschreiben oder der Patient kann die Injektionsvorrichtung auf eigene Rechnung kaufen, um ein Medikament in komfortablerer Weise injizieren zu können. Im Falle eines zu kühlenden Medikamentes kann dieses zudem separat zur Injektionsvorrichtung in gekühlter Weise gelagert und transportiert werden. Die Lagerung und der Transport der Injektionsvorrichtung selbst kann dagegen ohne Kühlung erfolgen. Der benötigte Kühlraum wird auf diese Weise lediglich durch das Medikament beziehungsweise durch die Außenabmessungen der Spritzen definiert, in denen das Medikament aufgenommen ist. Vor Verabreichung des Medikaments kann eine betreffende Spritze dann in eine in die Offenstellung verbrachte Injektionsvorrichtung eingesetzt werden. Anschließend wird dann das zweite äußere Gehäuseteil gegenüber dem ersten äußeren Gehäuseteil in die Schließstellung verbracht, in der sich die Injektionsvorrichtung dann in einem betriebsbereiten Zustand befindet. Zudem weist die Übertragungseinrichtung zwischen der Stößelaufnahme und dem Betätigungselement ferner eine Kopplungseinrichtung auf, die ein stößelaufnahmeseitiges Kopplungsmittel und ein betätigungselementseitiges Kopplungsgegenmittel aufweist, die in der Offenstellung getrennt ist. Auf diese Weise kann eine versehentliche Kraftbeaufschlagung der Spritze in der Offenstellung der äußeren Gehäuseteile verhindert werden. Hierbei ist es günstig, wenn das stößelaufnahmeseitige Kopplungsmittel und das betätigungselementeseitige Kopplungsgegenmittel in der Schließstellung in einen formschlüssigen Eingriff gebracht werden können, der in und entgegen der Injektionsrichtung wirkt. Durch eine derart ausgebildete Kopplungseinrichtung kann eine trennbare und dennoch stabile Kraftübertragung vom Betätigungselement auf die Stößelaufnahme gewährleistet werden.

In einer besonders vorteilhaften Ausführungsform sind die beiden äußeren Gehäuseteile zueinander sowohl in die Offenstellung als auch in die Schließstellung vorgespannt. Durch eine derartige bistabile Positionierung der beiden äußeren Gehäuseteile kann die Spritze in der Offenstellung besonders einfach eingesetzt werden, während die Spritze in der Schließstellung sicher und geschützt im Inneren der Injektionsvorrichtung aufgenommen ist.

Dabei ist es günstig, wenn das zweite äußere Gehäuseteil in der Schließstellung mit dem ersten äußeren Gehäuseteil dauerhaft oder lösbar verbunden werden kann. Auf diese Weise kann im Betrieb ein versehentliches Öffnen des Gehäuses vermieden werden. Hierbei ist das zweite äußere Gehäuseteil in der Schließstellung mit dem ersten äußeren Gehäuseteil beispielsweise dauerhaft fest verbunden. Auf diese Weise kann die Verwendung der Injektionsvorrichtung als Einwegprodukt sichergestellt werden. Dabei ist vorteilhafterweise zwischen dem ersten äußeren Gehäuseteil und dem zweiten äußeren Gehäuseteil eine unlösbare Schnappverbindung vorgesehen, wodurch nach erfolgter Verabreichung des Medikamentes mittels der als Einwegprodukt ausgelegten Injektionsvorrichtung ein Öffnen derselben wirksam verhindert werden kann. In einer hierzu alternativen Ausführungsform der Injektionsvorrichtung können das erste äußere Gehäuseteil und das zweite äußere Gehäuseteil in der Schließstellung durch eine auf das Gehäuse unlösbar aufschiebbare Fingeranlage verriegelt werden. Hierbei ermöglicht die separate Fingeranlage eine besonders einfache Herstellung der für die unlösbare Verriegelung benötigten Mittel. In einer weiteren alternativen Ausführungsform der Injektionsvorrichtung kann das zweite äußere Gehäuseteil in der Schließstellung mit dem ersten äußeren Gehäuseteil lösbar verbunden werden. Die lösbare Verbindung der beiden Gehäuseteile ermöglicht dabei eine mehrfache Verwendung der Injektionsvorrichtung. Vorteilhafterweise ist hierzu zwischen dem ersten äußeren Gehäuseteil und dem zweiten äußeren Gehäuseteil eine lösbare Schnappverbindung vorgesehen, wodurch in der Schließstellung eine selbsttätige und stabile Verbindung zwischen beiden Gehäuseteilen gewährleistet werden kann. Alternativ hierzu können das erste äußere Gehäuseteil und das zweite äußere Gehäuseteil in der Schließstellung durch eine Fingeranlage verriegelt werden, die auf das Gehäuse lösbar aufgeschoben werden kann. Eine derartige Fingeranlage ermöglicht dabei eine besonders einfache und stabile lösbare Verbindung der beiden äußeren Gehäuseteile. Sowohl bei der dauerhaften als auch bei der lösbaren Verriegelung der beiden Gehäuseteile durch die Fingeranlage, kann diese dabei derart mit der übrigen Injektionsvorrichtung verbunden oder an dieser geführt sein, dass sie auch vor und/oder nach der Verriegelung unverlierbar an der Injektionsvorrichtung gehalten ist.

Vorteilhafterweise ist zwischen dem ersten äußeren Gehäuseteil und dem zweiten äußeren Gehäuseteil ein Endanschlag ausgebildet, über den die beiden äußeren Gehäuseteile in der Offenstellung aneinander anlegbar sind. Mittels dieses Endanschlages kann bei einer Verlagerung der beiden äußeren Gehäuseteile in die Offenstellung eine Überbeanspruchung des Schwenklagers insbesondere in Form einer Materialüberdehnung verhindert werden.

In einer weiteren vorteilhaften Ausführungsform sind das erste äußere Gehäuseteil und das zweite äußere Gehäuseteil beim Verschwenken von der Offenstellung in die Schließstellung aneinander geführt, wodurch eine stabile Schwenkbewegung in die exakte Schließstellung gewährleistet ist. Dabei ist es günstig, wenn zwischen dem ersten äußeren Gehäuseteil und dem zweiten äußeren Gehäuseteil quer zur Schwenkrichtung wirkende Zugentlastungsmittel vorgesehen sind. Derartige Zugentlastungsmittel können dabei beispielsweise durch gegenseitig in Eingriff bringbare Elemente gebildet sein, mittels denen ein in Richtung einer Haupterstreckungsrichtung der Injektionsvorrichtung wirksamer Formschluss hergestellt werden kann. Auf diese Weise kann das zwischen den beiden äußeren Gehäuseteilen angeordnete Schwenklager durch die Zugentlastungsmittel im Betrieb vor Beschädigungen infolge einer äußeren Kraftbeaufschlagung geschützt werden. Zudem ist es von Vorteil, wenn das Betätigungselement in der Schließstellung an der Stößelaufnahme abgestützt ist. Hierdurch kann das Betätigungselement durch die Stößelaufnahme stabilisiert werden, um insbesondere hinsichtlich der während einer Anwendung auftretenden Krafteinwirkungen eine ausreichende Torsions- und Biegesteifigkeit aufzuweisen. Ferner ist es günstig, wenn die Stößelaufnahme einen Kontaktabschnitt zur Anlage an den Spritzenstößel aufweist, an dem seitliche Abstützmittel ausgeformt sind. Derartige seitliche Abstützmittel können zur Zentrierung des Spritzenstößels in der Stößelaufnahme dienen, um bei einer Benutzung der Injektionsvorrichtung eine exakte Beaufschlagung desselben durch die Stößelaufnahme zu gewährleisten.

Ferner ist es günstig, wenn das Schwenklager eine Sollbruchstelle ausbildet. Hierdurch ist es möglich, die Injektionsvorrichtung für eine vorgegebene Anzahl von Anwendungen auszulegen und das Erreichen dieser vorgegebenen Anzahl durch Abbrechen des Schwenklagers anzuzeigen. Hierdurch ist es möglich die Funktionsfähigkeit der Injektionsvorrichtung zu beenden, bevor beispielsweise Beeinträchtigungen an der Steuerungsanordnung auftreten können.

Zudem ist es günstig wenn eine Betätigungssicherung vorgesehen ist, die in der Offenstellung eine Verlagerung des Betätigungselementes verhindert. Hierdurch können sowohl Fehlbedienungen der Injektionsvorrichtungen als auch Verletzungen der bedienende Person verhindert werden. Vorteilhafterweise weist die Betätigungssicherung hierbei einen Verriegelungsmechanismus auf, der in der Schließstellung der Gehäuseteile entriegelt ist. Durch einen derartigen Verriegelungsmechanismus kann die Betätigungssicherung durch alleiniges verlagern der äußeren Gehäuseteile in die Schließstellung beziehungsweise durch das Anbringen der Fingeranlage selbsttätig entriegelt werden.

Ferner sind die Spritzenaufnahme und die Stößelaufnahme am ersten äußeren Gehäuseteil und das Betätigungselement und das Zahnradgetriebe am zweiten äußeren Gehäuseteil gehalten.

In einer besonders bevorzugten Ausführungsform der Injektionsvorrichtung sind ferner Adaptermittel vorgesehen, mittels denen die Spritzenaufnahme und/oder die Stößelaufnahme an unterschiedliche Spritzen angepasst werden kann. Durch derartige Adaptermittel ist es dabei insbesondere möglich, die Injektionsvorrichtung jeweils an Spritzen mit unterschiedlichen Längen anzupassen. Alternativ oder zusätzlich hierzu können die Spritzenaufnahme und/oder die Stößelaufnahme austauschbar ausgebildet sein. Auf diese Weise können für unterschiedliche Spritzen entsprechend dimensionierte Spritzenaufnahmen beziehungsweise Stößelaufnahmen verwendet werden.

In einer weiteren vorteilhaften Ausführungsform der Injektionsvorrichtung sind zwischen den äußeren Gehäuseteilen und der Spritzenaufnahme und/oder der Stößelaufnahme Codierungsmittel vorgesehen, die eine Ausgangsstellung definieren, in der die äußeren Gehäuseteile in die Schließstellung verbracht werden können. Auf diese Weise kann sichergestellt werden, dass die äußeren Gehäuseteilen nur dann in die Schließstellung verlagert werden können, wenn sich die Spritzenaufnahme und/oder die Stößelaufnahme in der besagten Ausgangsstellung befinden.

Ferner ist zwischen dem ersten äußeren Gehäuseteil und dem zweiten äußeren Gehäuseteil ein Sicherungsmechanismus vorgesehen, mittels dem verhindert werden kann, dass die beiden äußeren Gehäuseteile während des Betriebs der Injektionsvorrichtung in die Offenstellung verbracht werden können. Hierdurch kann eine sichere Verwendung der Injektionsvorrichtung gewährleistet werden. Dabei weist der Sicherungsmechanismus vorteilhafterweise an einem der äußeren Gehäuseteile ein Hintergreifelement auf, mittels dem ein Rand einer Sicherungsnut des jeweils anderen Gehäuseteils hintergriffen werden kann. Dabei ist es günstig, wenn die Sicherungsnut an der Stößelaufnahme eingelassen und das Hintergreifelement betätigungselementseitige Codierungsmittel ausbildet, wobei die Sicherungsnut eine Eintrittsöffnung aufweist, die in der Ausgangsstellung der Spritzenaufnahme auf Höhe der Codierungsmittel angeordnet ist. Hierdurch kann ein Öffnen der äußeren Gehäuseteile während der Anwendung der Injektionsvorrichtung wirksam verhindert werden.

Vorteilhafterweise ist zwischen der Spritzenaufnahme und der Stößelaufnahme ein lösbarer Verbindungsmechanismus angeordnet, der ein Einrastelement aufweist, das in einer Einstechstellung und in einer Rückholstellung mit einer jeweiligen Einrastaufnahme in Eingriff gebracht werden kann. Auf diese Weise kann die Spritzenaufnahme sowohl während eines Einstechhubes als auch während eines Rückholhubes sicher mit der Stößelaufnahme bewegungsgekoppelt werden. Dabei ist es günstig, wenn das Einrastelement durch einen über einen elastischen Arm an der Stößelaufnahme gehaltenen Rasthaken gebildet ist, der über ein an der Spritzenaufnahme ausgeformtes Steuerungsprofil zwangsgesteuert ist. Auf diese Weise kann die Koppelung und Entkoppelung der Stößelaufnahme mit der Spritzenaufnahme im Verlauf der verschiedenen Hube während einer Anwendung exakt gesteuert werden. Vorteilhafterweise ist der elastische Arm dabei in der Einstechstellung spannungsfrei gehalten. Hierdurch kann die Injektionsvorrichtung auch in einem bereits betriebsbereiten Zustand über einen längeren Zeitraum hinweg gehalten beziehungsweise gelagert werden, ohne dass es hierbei zu einer Materialermüdung des elastischen Armes kommen kann.

In einer weiteren vorteilhaften Ausführungsform der Injektionsvorrichtung ist ein Verlagerungsmechanismus vorgesehen, mittels dem die Stößelaufnahme bei Verlagerung der Gehäuseteile in die Offenstellung selbsttätig in die Ausgangsstellung verbracht werden kann. Durch einen derartigen Verlagerungsmechanismus kann die für eine mehrfache Verwendung ausgelegte Injektionsvorrichtung allein durch Öffnen der äußeren Gehäuseteile in die Ausgangsstellung verbracht werden, um nach dem Austausch der verbrauchten Spritze durch eine neue Spritze und dem erneuten Verlagern der äußeren Gehäuseteile in die Schließstellung wieder in einen betriebsbereiten Zustand verbracht werden. Vorteilhafterweise weist der Verlagerungsmechanismus dabei eine Federeinrichtung auf, die die Stößelaufnahme in der Offenstellung in die Ausgangsstellung vorspannt. Gleichzeitig werden hierbei auch das stößelaufnahmeseitige Kopplungsmittel und das Betätigungselement mit dem betätigungselementseitigen Kopplungsgegenmittel in die Ausgangsstellung verbracht. Hierdurch kann die Injektionsvorrichtung in besonders komfortabler Weise für einen erneuten Anwendungsvorgang vorbereitet werden. Dabei ist es günstig, wenn zwischen dem Gehäuse und der Stößelaufnahme Festlegemittel vorgesehen sind, mittels denen wenigstens die Stößelaufnahme in der Ausgangsstellung gegenüber dem Gehäuse festgelegt werden kann. Hierdurch kann die Injektionsvorrichtung zuverlässig in der Ausgangsstellung gehalten werden.

In einer weiteren vorteilhaften Ausführungsform der Injektionsvorrichtung ist das Betätigungselement mit einer Dämpfungseinrichtung gekoppelt. Hierdurch kann eine Verlagerungsgeschwindigkeit des Betätigungselementes während einer Anwendung in geeigneter Weise begrenzt werden. Dabei weist die Dämpfungseinrichtung einen linear wirkenden Luftdämpfer auf, bei dem ein mit dem Betätigungselement bewegungsgekoppelter Verdrängungskörper entlang eines Luftraumes verlagert werden kann. Hierdurch ist die Dämpfungseinrichtung in besonders kostengünstiger Weise herstellbar.

Ferner ist es günstig, wenn eine Endlagenverriegelung vorgesehen ist, mittels der das Betätigungselement in einer bei Abschluss eines Injektionsvorganges eingenommenen Endlage festgelegt werden kann. Hierdurch kann verhindert werden, dass das Betätigungselement nach Abschluss eines Injektionsvorganges versehentlich erneut verlagert wird. Hierbei kann die Endlagenverriegelung durch Verlagerung der äußeren Gehäuseteile in die Offenstellung in eine Freigabestellung verbracht werden. Auf diese Weise kann das Betätigungselement nach Öffnen des Gehäuses zur Herstellung eines betriebsbereiten Zustandes der Injektionsvorrichtung wieder in seine Ausgangsstellung verlagert werden.

In den Figuren ist eine beispielhafte Ausführungsform der Erfindung dargestellt. Es zeigen:
- Figur 1: eine perspektivische Ansicht einer nicht unter die beanspruchte Erfindung fallende Injektionsvorrichtung in einer Offenstellung,
- Figur 2A: eine perspektivische Ansicht der Injektionsvorrichtung nach Figur 1 mit aufgenommener Spritze,
- Figur 2B: eine Draufsicht auf die Injektionsvorrichtung nach Figur 1,
- Figur 2C: eine geschnittene Ansicht der Injektionsvorrichtung in Ebene IIC-IIC aus Figur 2B,
- Figur 2D: eine geschnittene Ansicht der Injektionsvorrichtung in Ebene IID-IID aus Figur 2B,
- Figur 3A: eine perspektivische Ansicht eines Gehäuses der Injektionsvorrichtung nach Figur 1 in der Offenstellung,
- Figur 3B: eine perspektivische Ansicht der Injektionsvorrichtung nach Figur 1 in einer Schließstellung,
- Figur 3C: eine perspektivische Ansicht der Injektionsvorrichtung nach Figur 3B mit einem alternativen Beispiel eines Schwenklagers,
- Figur 3D: eine vergrößerte geschnittene Ansicht durch das Schwenklager nach Figur 3C,
- Figur 3E: eine Seitenansicht der Injektionsvorrichtung nach Figur 1 beim Verbringen in die Schließstellung,
- Figur 3F: eine vergrößerte Ansicht von Führungsmitteln der Injektionsvorrichtung nach Figur 3 beim Verbringen in die Schließstellung,
- Figur 4A: eine perspektivische Ansicht der Injektionsvorrichtung nach Figur 1 in einer Ausgangsstellung,
- Figur 4B: eine perspektivische Ansicht der Injektionsvorrichtung nach Figur 1 mit gelöster Fingeranlage,
- Figur 4C: einen Längsschnitt durch die Injektionsvorrichtung nach Figur 4A,
- Figur 4D: eine vergrößerte Ansicht einer Betätigungssicherung der Injektionsvorrichtung aus Figur 4C,
- Figur 5A: eine perspektivische Ansicht der Injektionsvorrichtung nach Figur 4A bei entferntem Deckel nach Durchführung eines Einstechhubes,
- Figur 5B: eine Draufsicht auf die Injektionsvorrichtung nach Figur 5A,
- Figur 5C: eine geschnittene Ansicht der Injektionsvorrichtung in Ebene VC-VC aus Figur 5B,
- Figur 6A: eine perspektivische Ansicht der Injektionsvorrichtung nach Figur 4A bei entferntem Deckel nach Durchführung eines Injektionshubes,
- Figur 6B: eine Draufsicht auf die Injektionsvorrichtung nach Figur 6A,
- Figur 6C: eine geschnittene Ansicht der Injektionsvorrichtung in Ebene VIC-VIC aus Figur 6B,
- Figur 7A: eine perspektivische Ansicht der Injektionsvorrichtung nach Figur 4A bei entferntem Deckel nach Durchführung eines Rückholhubes,
- Figur 7B: eine Draufsicht auf die Injektionsvorrichtung nach Figur 7A,
- Figur 7C: eine geschnittene Ansicht der Injektionsvorrichtung in Ebene VIIC-VIIC aus Figur 7B,
- Figur 8A: eine perspektivische Ansicht der Injektionsvorrichtung in erfindungsgemäßer Ausführungsform in der Offenstellung,
- Figur 8B: eine Draufsicht auf die Injektionsvorrichtung nach Figur 8A,
- Figur 8C: eine geschnittene Ansicht der Injektionsvorrichtung in Ebene VIIIC-VIIIC aus Figur 8B,
- Figur 8D: eine geschnittene Ansicht der Injektionsvorrichtung in Ebene VIIID-VIIID aus Figur 8B,
- Figur 8E: eine geschnittene Ansicht der Injektionsvorrichtung in Ebene VIIIE-VIIIE aus Figur 8B,
- Figur 8F: eine vergrößerte Ansicht von Festlegemitteln der Injektionsvorrichtung aus Figur 8E,
- Figur 9A: eine perspektivische Ansicht der Injektionsvorrichtung nach Figur 8A in der Schließstellung,
- Figur 9B: eine geschnittene Ansicht einer Dämpfungseinrichtung der Injektionsvorrichtung aus Figur 9A und
- Figur 9C: eine geschnittene Ansicht eines Verlagerungsmechanismus der Injektionsvorrichtung aus Figur 9A.

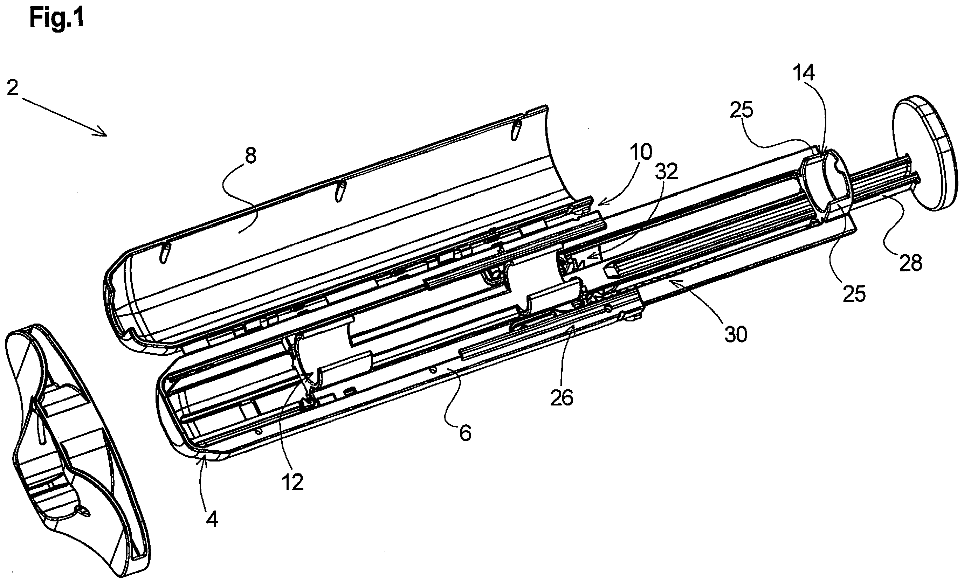

Die Figuren 1 bis 7 zeigen einen prinzipiellen Ablauf eines Injektionsvorgangs anhand eines Ausführungsbeispiels, das nicht unter die beanspruchte Erfindung fällt.

Fig. 1 zeigt eine Injektionsvorrichtung 2 mit einem Gehäuse 4, dass ein erstes äußeres Gehäuseteil 6 und ein zweites äußeres Gehäuseteil 8 aufweist. Die beiden äußeren Gehäuseteilen 6, 8 sind dabei über ein Schwenklager 10 miteinander verbunden, mittels dem sie zueinander zwischen einer Offenstellung und einer Schließstellung verlagert werden können.

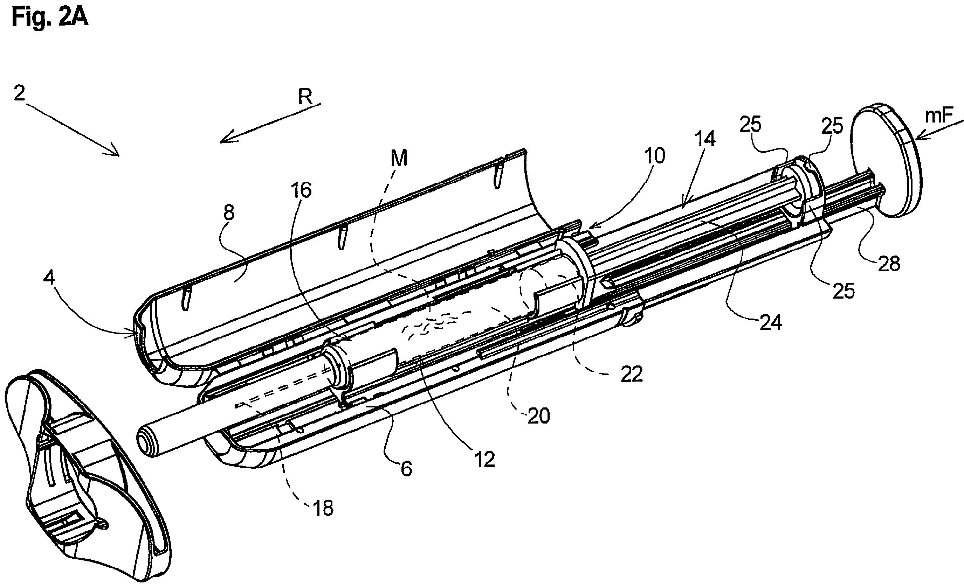

In der dargestellten Offenstellung wird hierbei der Zugriff auf eine Spritzenaufnahme 12 und eine Stößelaufnahme 14 der Injektionsvorrichtung 2 freigegeben. In diesen kann, wie insbesondere aus Fig. 2A zu entnehmen ist, eine mit einem zu verabreichenden Medikament M gefüllte Spritze 16 aufgenommen werden, die eine Injektionsnadel 18, eine Aufnahmekammer 20, einen Kolben 22 und einen Spritzenstößel 24 aufweist. Die Aufnahmekammer 20 wird hierzu in der Spritzenaufnahme 12 und der Spritzenstößel 24 in der Stößelaufnahme 14 festgelegt. Hierzu bildet die Stößelaufnahme 14 durch seitliche Abstützmittel 25 einen Kontaktabschnitt, in dem das freie Ende des Spritzenstößels 24 in selbstzentrierender Weise aufgenommen wird.

Zur Durchführung eines gesteuerten Anwendungsablaufes, bei dem die Spritze 16 ausgepresst und das Medikament M einem Patienten verabreicht wird, weist die Injektionsvorrichtung 2 eine insgesamt mit 26 bezeichnete Steuerungsanordnung auf. Diese ermöglicht durch alleinige Beaufschlagung eines Betätigungselementes 28 mit einer manuellen Antriebskraft mF in eine Injektionsrichtung R die Durchführung eines Einstechhubes, eines Injektionshubes sowie eines Rückholhubes.

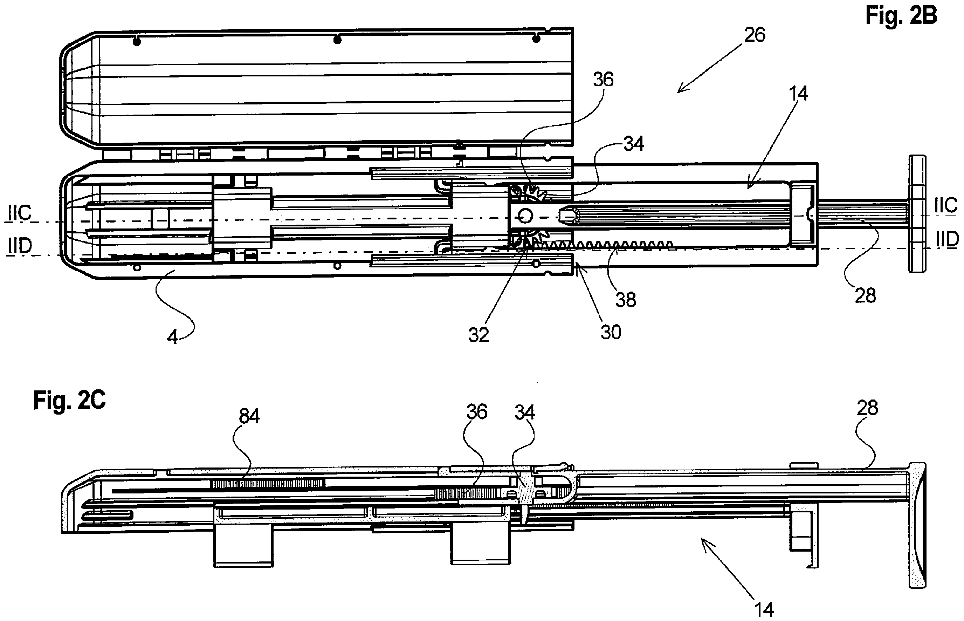

Hierzu weist die Steuerungsanordnung 26 eine Übertragungseinrichtung 30 mit einem Zahnradgetriebe 32 auf, welches das Betätigungselement 28 mit der Stößelaufnahme 14 koppelt, wie aus Fig. 2B und 2C zu entnehmen ist. Hierdurch kann die Stößelaufnahme 14 in Abhängigkeit von einer Relativbewegung des Betätigungselementes 28 gegenüber dem Gehäuse 4 angetrieben werden. Das Zahnradgetriebe 32 weist hierzu ein am Betätigungselement 28 verdrehbar gelagertes Zahnrad 34 auf, das sowohl mit wenigstens einer gehäuseseitigen Verzahnung 36 als auch mit einer stößelaufnahmeseitigen Verzahnung 38 in kämmenden Eingriff gebracht werden kann.

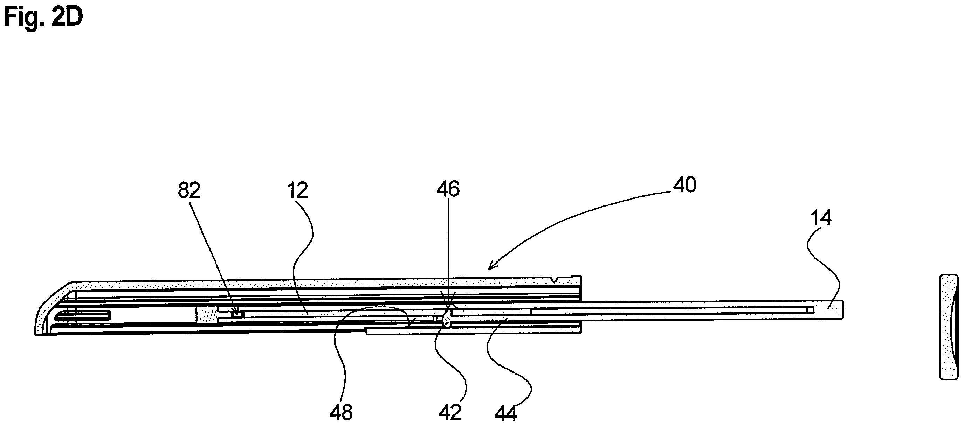

Ferner weist die Steuerungsanordnung 26 einen Verbindungsmechanismus 40 auf, der aus Figur 2D zu entnehmen ist. Bei diesem ist ein Einrastelement in Form eines Rasthakens 42 vorgesehen, der über einen elastischen Arm 44 an der Stößelaufnahme 14 gehalten ist. Dieser Rasthaken 42 steht in der dargestellten Ausgangsstellung in einem spannungsfreien Zustand in Eingriff mit einer ersten Einrastaufnahme 46 der Spritzenaufnahme 12 und liegt dabei an einer Innenseite 48 des Gehäuses 4 an, das als Steuerungsprofil fungiert.

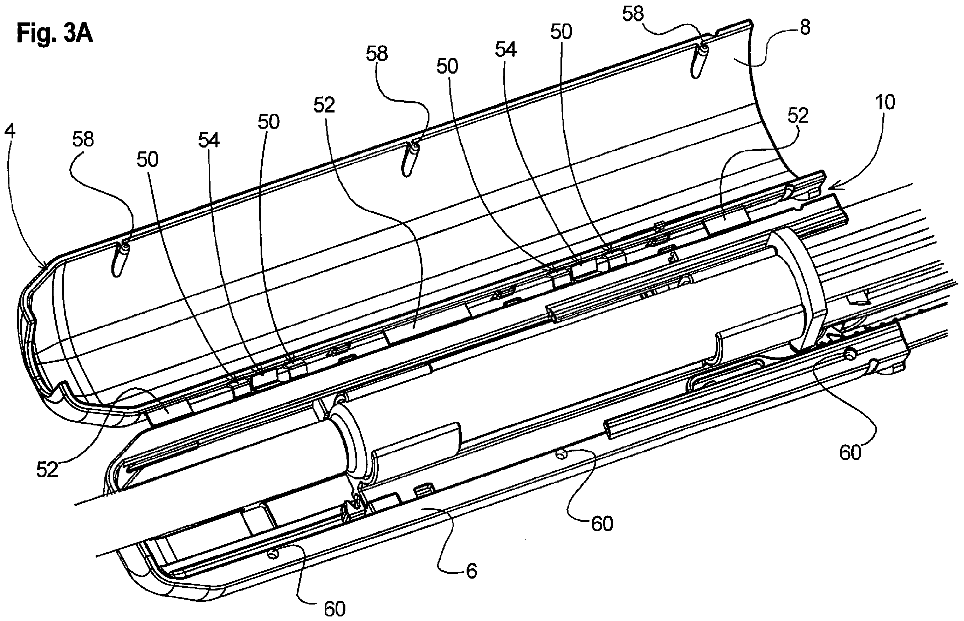

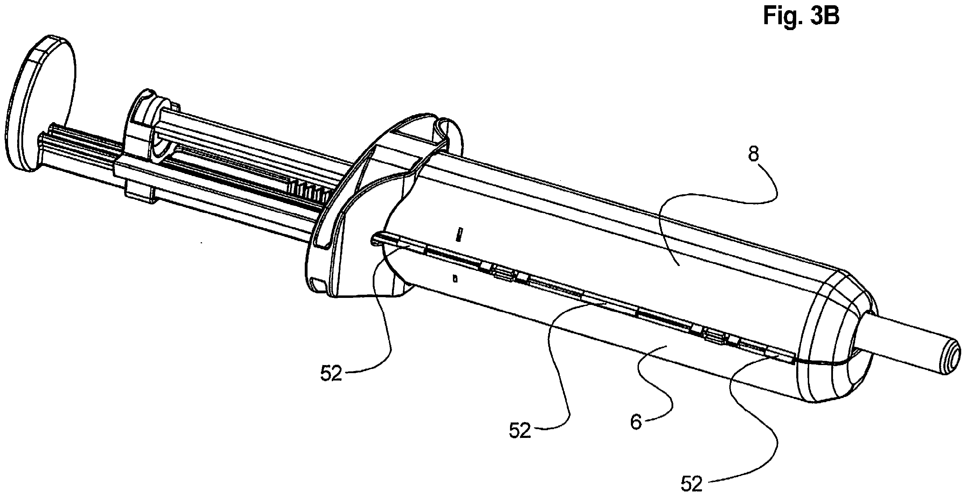

Wie insbesondere aus der vergrößerten Darstellung des Schwenklagers 10 in Figur 3A zu entnehmen ist, sind zwischen dem ersten äußeren Gehäuseteil 6 und dem zweiten äußeren Gehäuseteil 8 Vorspannmittel 50 vorgesehen, mittels denen die beiden äußeren Gehäuseteile 6, 8 sowohl in die dargestellte Offenstellung als auch in die Schließstellung vorgespannt werden können. Die Vorspannmittel 50 sind hierbei beispielhaft durch federnde Verbindungselemente in Form von Schmetterlingsscharnieren gebildet. Alternativ oder zusätzlich zu diesen können zwischen dem ersten äußeren Gehäuseteil 6 und dem zweiten äußeren Gehäuseteil 8 zudem einfache Filmscharniere 52 vorgesehen werden, wie in Fig. 3B beispielhaft dargestellt. In jedem Fall sind hierbei die beiden äußeren Gehäuseteile 6, 8 fest miteinander verbunden beziehungsweise einteilig ausgebildet. Dabei ist es auch möglich, das Schwenklager 10 derart auszubilden, dass es als Sollbruchstelle fungiert, das nach einer vorbestimmten Anzahl von Öffnungs- beziehungsweise Schließvorgängen des Gehäuses bricht, um eine Abnutzung der Injektionsvorrichtung 2 insgesamt anzuzeigen.

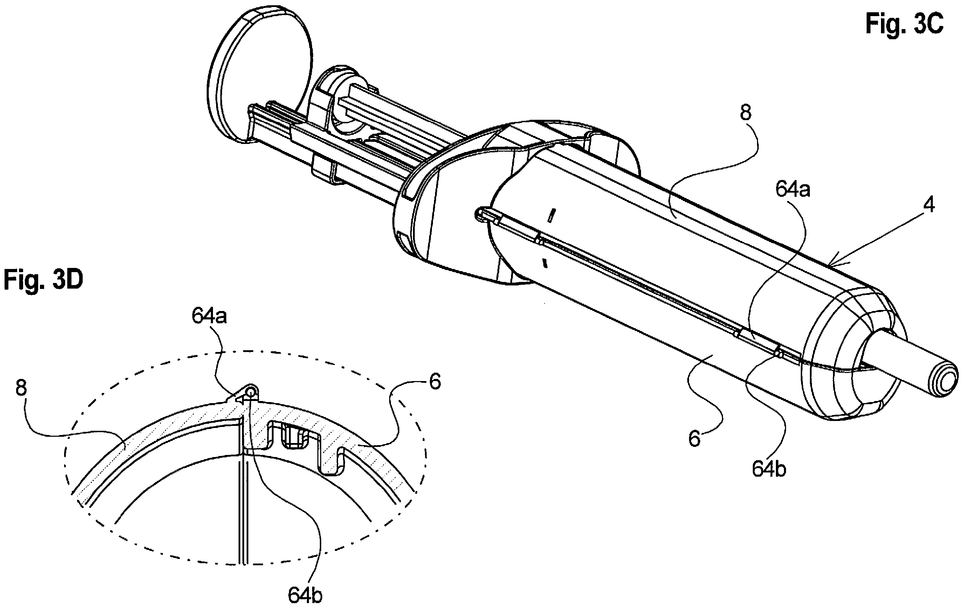

Alternativ hierzu kann das Schwenklager 10 auch durch miteinander verrastbare Scharniermittel 64a, 64b gebildet sein, wie in Figur 3C und 3D dargestellt. Hierdurch ist es möglich, die beiden äußeren Gehäuseteile 6, 8 getrennt voneinander herzustellen und anschließend miteinander zu verrasten, um das Gehäuse 4 auszubilden.

In jedem Fall können, wie aus Fig. 3A ferner zu entnehmen ist, am Schwenklager 10 zusätzliche Anschlagsmittel 54 zur Ausbildung eines Endanschlages vorgesehen sein, durch die eine Überbeanspruchung des Schwenklagers 10 insbesondere beim Verschwenken in die Offenstellung verhindert werden kann.

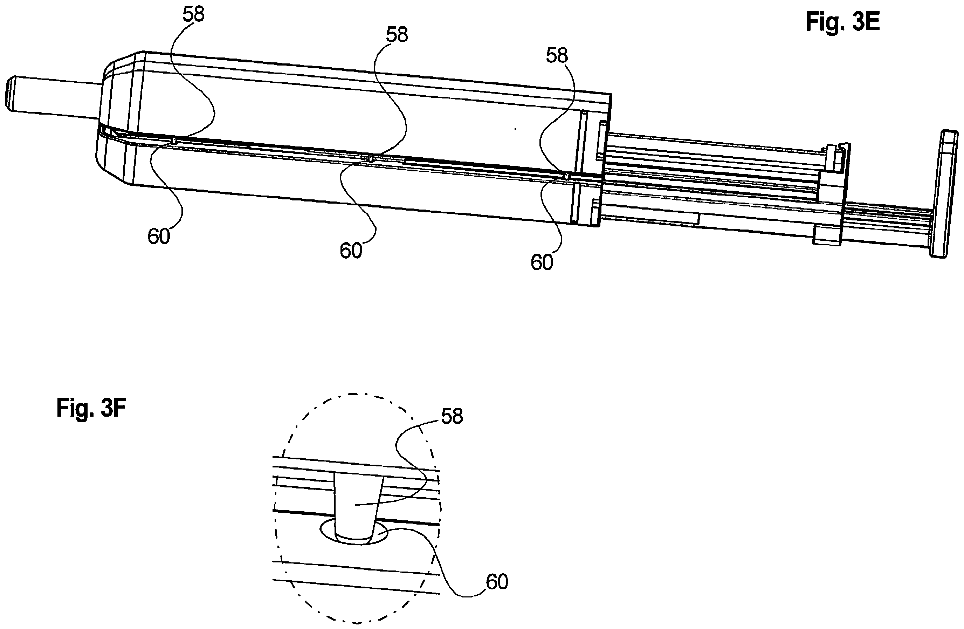

Darüber hinaus sind in das erste äußere Gehäuseteil 6 mehrere Führungsaufnahmen 60 eingelassen. In hierzu angepassten Positionen sind an dem zweiten äußeren Gehäuseteil 8 zudem Führungsstifte 58 vorgesehen. Wie aus Fig. 3E und 3F zu entnehmen ist, werden diese beim Schließen des Gehäuses 4 in Führungsaufnahmen 60 eingeführt. In der Schließstellung des Gehäuses 4 wirken diese Führungsaufnahmen 60 dann mit den darin aufgenommenen Führungsstiften 58 quer zur Schwenkrichtung zudem als Zugentlastungsmittel.

In jedem Fall ist hierbei vorgesehen, dass die beiden äußeren Gehäuseteile 6, 8 in dem Ausführungsbeispiel der Injektionsvorrichtung 2 nach den Figuren 1 bis 3 in der Schließstellung unlösbar miteinander verriegelt werden können. Durch die hierdurch erzielte dauerhafte Verbindung zwischen beiden äußeren Gehäuseteilen 6, 8 kann eine Verwendung der Injektionsvorrichtung 2 als reines Einwegprodukt gewährleistet werden.

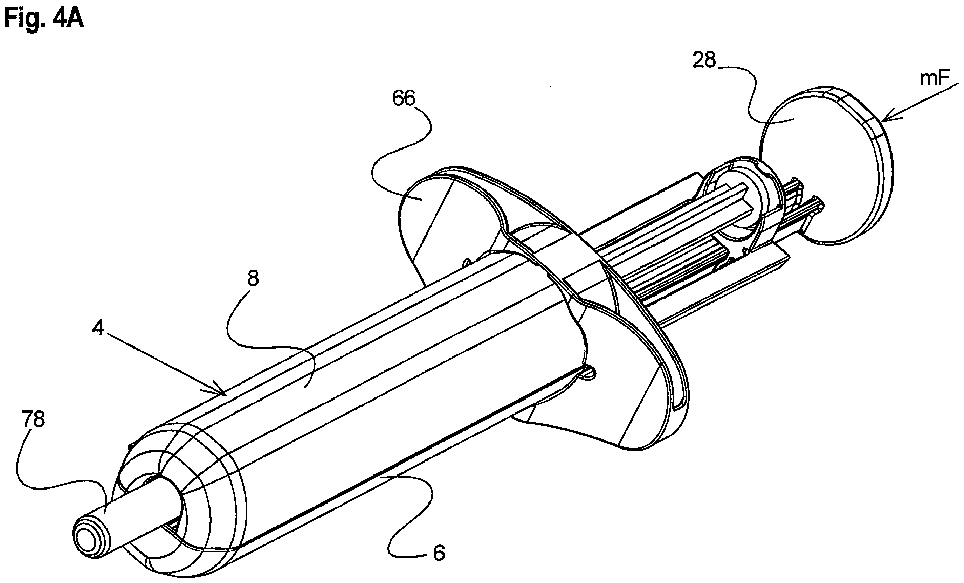

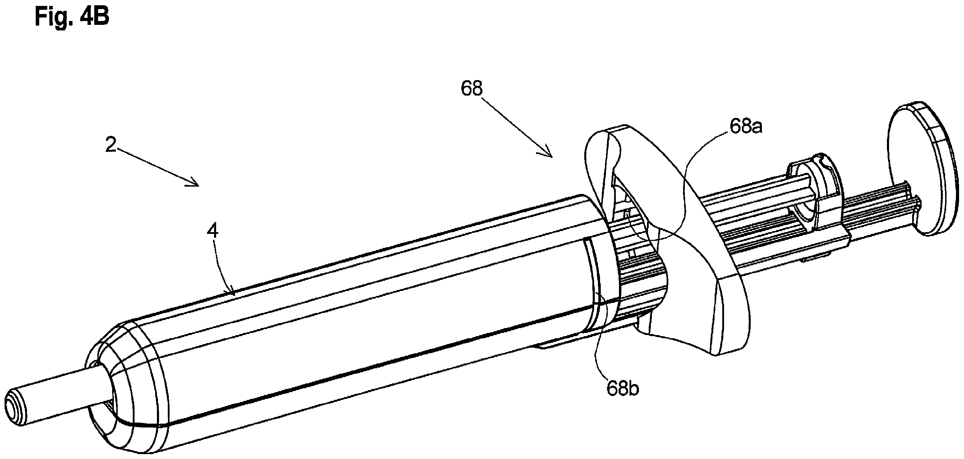

Hierzu kann beispielsweise eine Fingeranlage 66 vorgesehen sein, die in der Schließstellung auf das Gehäuse 4 aufgeschoben wird, und dabei die beiden äußeren Gehäuseteile 6, 8 ringförmig umschließt, wie in Figur 4A dargestellt. Um hierbei eine dauerhaft feste Verbindung der beiden äußeren Gehäuseteile 6, 8 zu gewährleisten, ist zwischen der Fingeranlage 66 und dem Gehäuse 4 beispielhaft eine unlösbare Schnappverbindung 68 vorgesehen. Diese kann, wie in Fig. 4B dargestellt, beispielsweise anlageseitig Rastelemente 68a aufweisen, die mit gehäuseseitigen Rastaufnahmen 68b verrastbar sind oder umgekehrt. Alternativ hierzu können auch direkt zwischen den beiden äußeren Gehäuseteilen 6, 8 unlösbare Verbindungsmittel vorgesehen sein (nicht dargestellt).

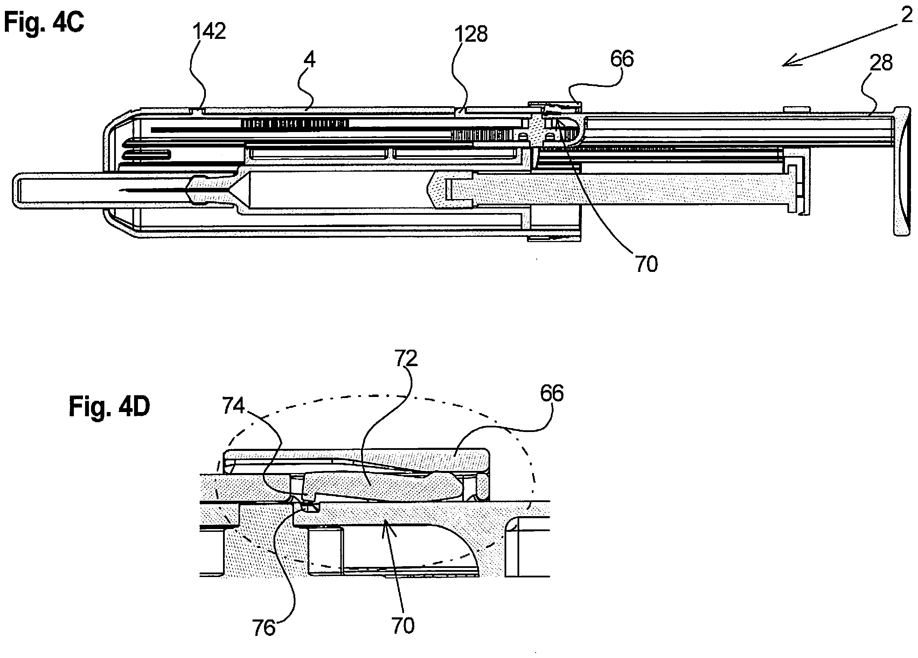

Wie aus den Figuren 4C und 4D ferner zu entnehmen ist, weist die Injektionsvorrichtung 2 eine Betätigungssicherung 70 auf, mittels der eine Verlagerung des Betätigungselementes 28 in der Offenstellung des Gehäuses 4 blockiert werden kann. Die Betätigungssicherung 70 weist hierzu einen verschwenkbaren Hebel 72 auf, an dem eine Eingriffsnase 74 ausgebildet ist, die in eine Eingriffsposition mit einer in das Betätigungselement 28 eingelassenen Eingriffsaufnahme 76 vorgespannt ist.

Durch das Aufschieben der Fingeranlage 66 auf das Gehäuse 4 wird der Hebel 72 derart verschwenkt, dass die Eingriffsnase 74 außer Eingriff mit der Eingriffsaufnahme 76 kommt und die Injektionsvorrichtung 2 somit betriebsbereit ist. Nach Entfernen einer Schutzkappe 78 von der Injektionsnadel 18 und Anlage des stirnseitigen Endes der Injektionsvorrichtung 2 an einem Patienten kann somit ein Injektionsvorgang durch Beaufschlagung des Betätigungselementes 28 mit der manuellen Kraft mF gemäß Figur 4A gestartet werden, wie beispielsweise durch einen Daumen des Patienten (nicht dargestellt).

Um hierbei einen vorbestimmten Schwellenwert für die Kraft mF festlegen zu können, ab dessen Erreichen der Injektionsvorgang erst gestartet wird, kann wie in Fig. 4C dargestellt ein Startkrafterzeuger 128 in Form eines elastisch verschwenkbaren Rasthakens vorgesehen werden, der bei Erreichen dieses Schwellenwertes außer Eingriff mit dem Gehäuse 4 kommt und dadurch erst die Verlagerung des Betätigungselementes 28 ermöglicht.

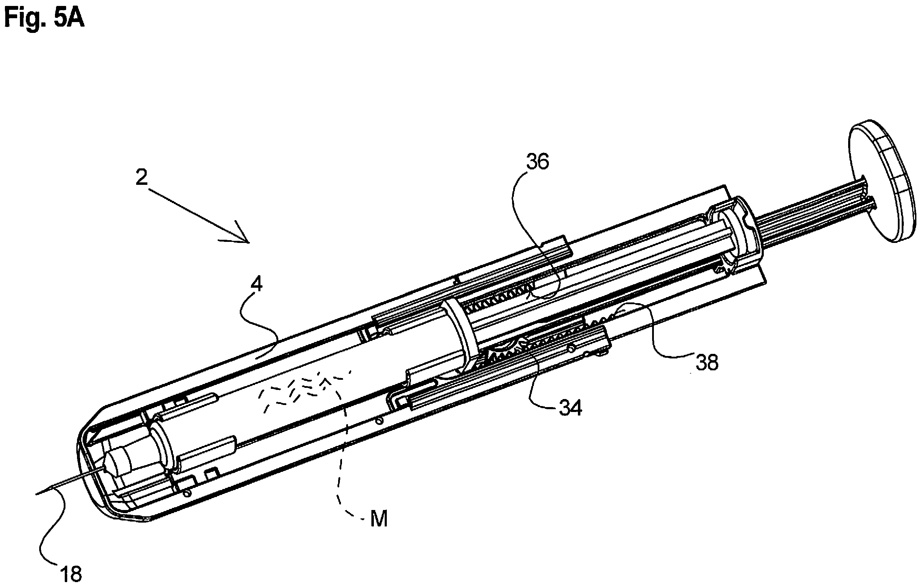

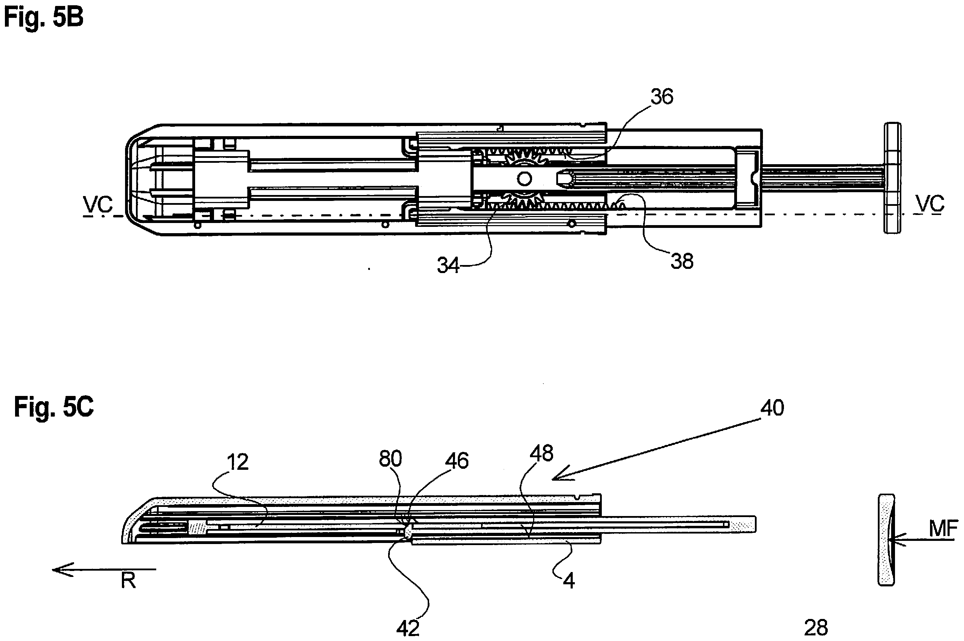

Hierdurch erfolgt zunächst der Einstechhub, bei dem das Zahnrad 34 sowohl mit der gehäuseseitigen Verzahnung 36 als auch mit der stößelaufnahmeseitigen Verzahnung 38 kämmt und durch den die Injektionsvorrichtung 2 in die in den Figuren 5A bis 5C gezeigte Position verlagert wird. In dieser ragt die Injektionsnadel 18 aus dem Gehäuse 4 heraus. Gleichzeitig ist der Rasthaken 42 des Verbindungsmechanismus 40 nun so weit verlagert dass er nicht mehr an der Innenseite 48 des Gehäuses 4 anliegt. Durch die weitere Beaufschlagung des Betätigungselementes 28 mit der manuellen Kraft mF kann er nun in Folge einer an ihm ausgebildeten stirnseitigen Schräge 80, die gegen die Spritzenaufnahme 12 drückt, seitlich aus der Einrastaufnahme 46 herausgeschwenkt und gegenüber der Spritzenaufnahme 12 weiter in Injektionsvorrichtung R verlagert werden.

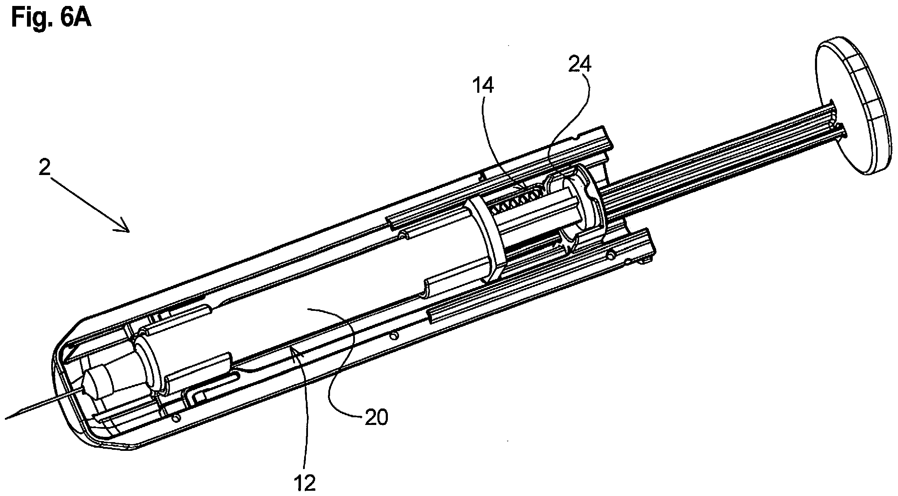

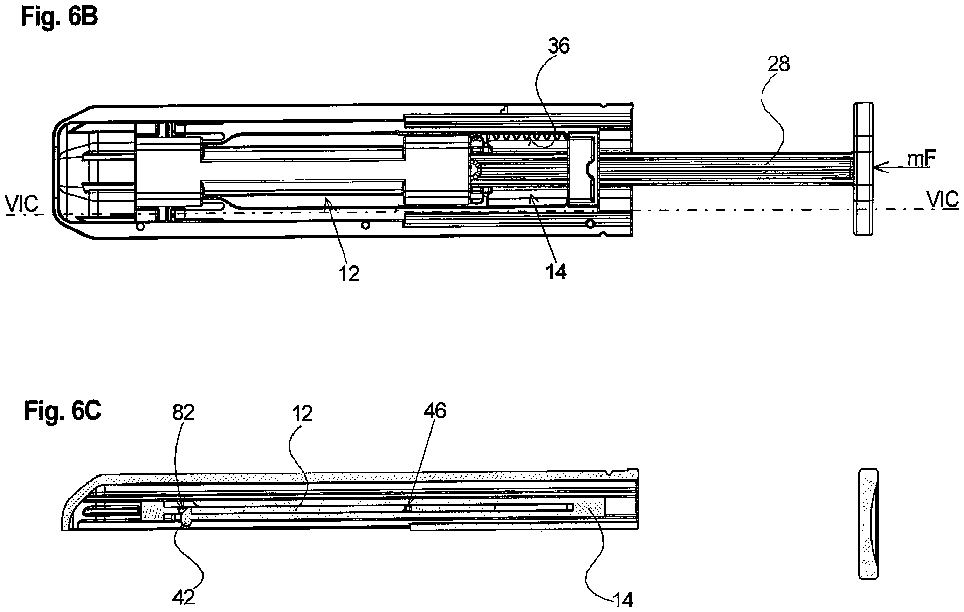

Hierdurch erfolgt nun der Injektionshub, bei dem die Stößelaufnahme 14 mit dem darin aufgenommenen Spritzenstößel 24 zum Auspressen des Medikamentes M gegenüber der Spritzenaufnahme 12 mit der darin aufgenommenen Aufnahmekammer 20 verlagert wird, bis die in den Figuren 6A bis 6C dargestellte Position der Injektionsvorrichtung 2 erreicht ist. In dieser gelangt das Zahnrad 34 außer Eingriff mit der gehäuseseitigen Verzahnung 36, so dass die weitere Verlagerung des Betätigungselementes 28 nicht weiter auf die Spritzenaufnahme 12 und/oder die Stößelaufnahme 14 übertragen wird und folglich ein Leerhub ausgeführt wird. Zudem ist der Rasthaken 42 des Verbindungsmechanismus 40 am Ende des Injektionshubes auf Höhe einer zweiten Einrastaufnahme 82 der Spritzenaufnahme 12 angeordnet, mit der er infolge seiner elastischen Rückstellkräfte in Eingriff gelangt.

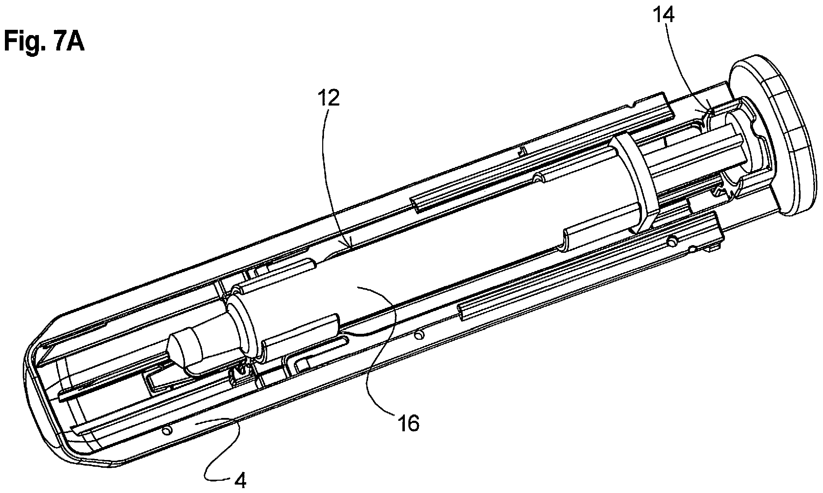

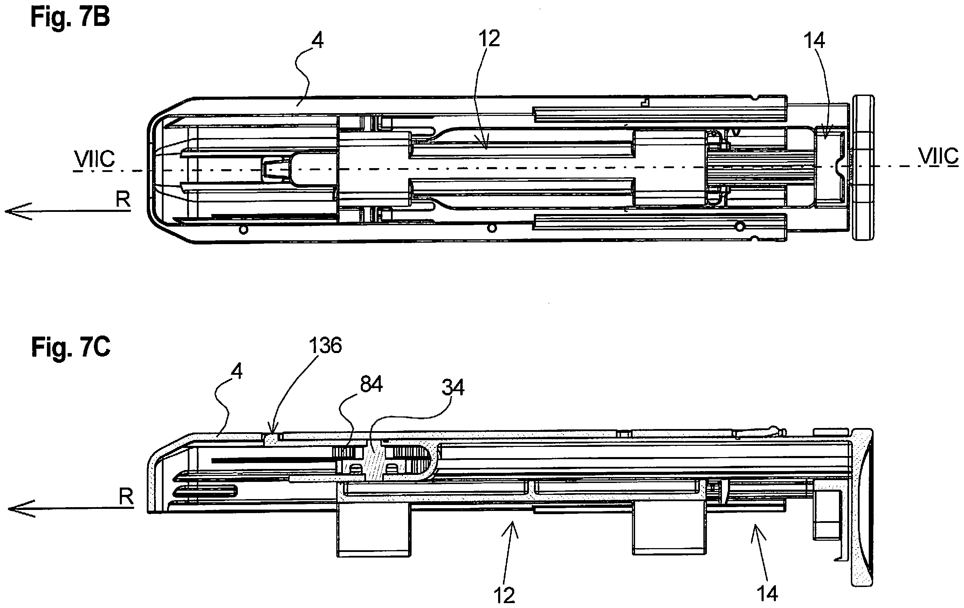

Durch die weitere Verlagerung des Betätigungselementes 28 in Injektionsrichtung R gelangt das Zahnrad 34 nach durchlaufen des Leerhubes in kämmenden Eingriff mit einer zweiten gehäuseseitigen Verzahnung 84, die an einer der ersten gehäuseseitigen Verzahnung 36 gegenüberliegenden Seite angeordnet ist (siehe Figur 2C). In Folge hiervon wird das Zahnrad 34, das gleichzeitig mit der stößelaufnahmeseitigen Verzahnung 38 kämmt, in entgegengesetzter Richtung wie zuvor gedreht und dadurch sowohl die Stößelaufnahme 14 als auch die mit ihr über den Rasthaken 42 und die Einrastaufnahme 82 bewegungsgekoppelte Spritzenaufnahme 12 entgegen der Injektionsvorrichtung R verlagert. Dieser Rückholhub erfolgt dabei gemäß den Figuren 7A bis 7C soweit, bis die nun ausgepresste Spritze 16 wieder vollständig innerhalb des Gehäuses 4 angeordnet ist. In dieser Endlage kann der durch einen Rasthaken gebildete Startkrafterzeuger 128 mit einer Verriegelungsausnehmung 142 gemäß Fig. 4C verrasten und somit wieder gegenüber dem Gehäuse festgelegt werden, um eine erneute Aktivierung der Injekitonsvorrichtung über das Betätigungselement zu vermeiden.

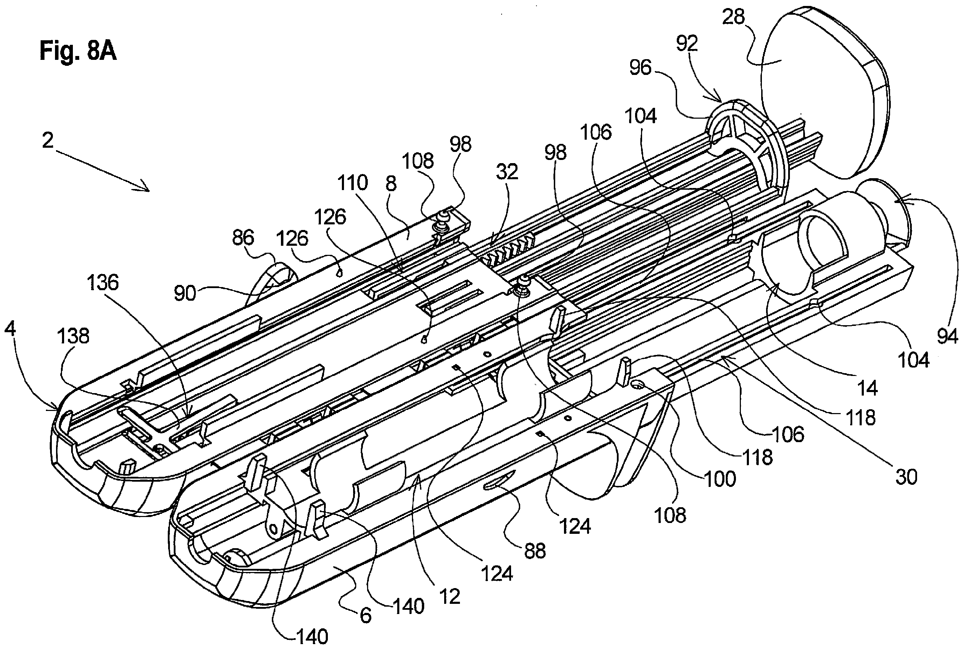

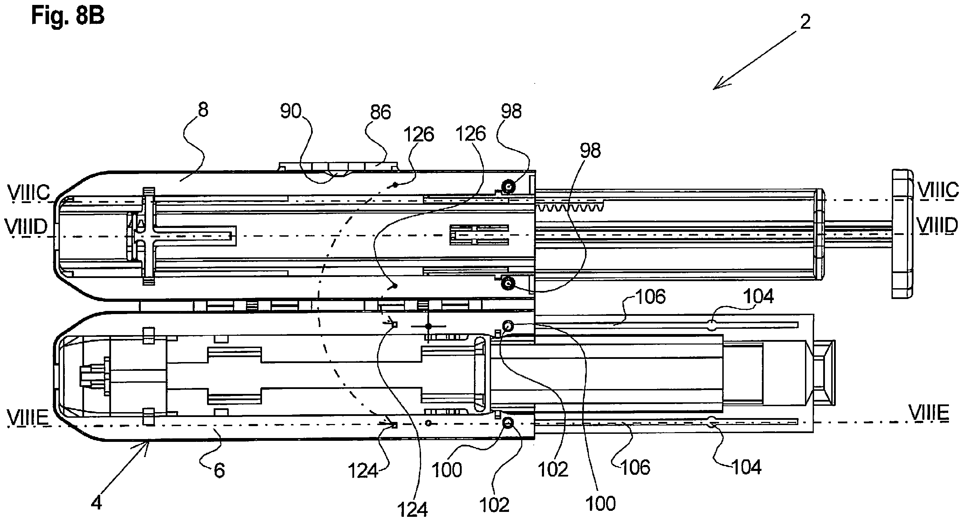

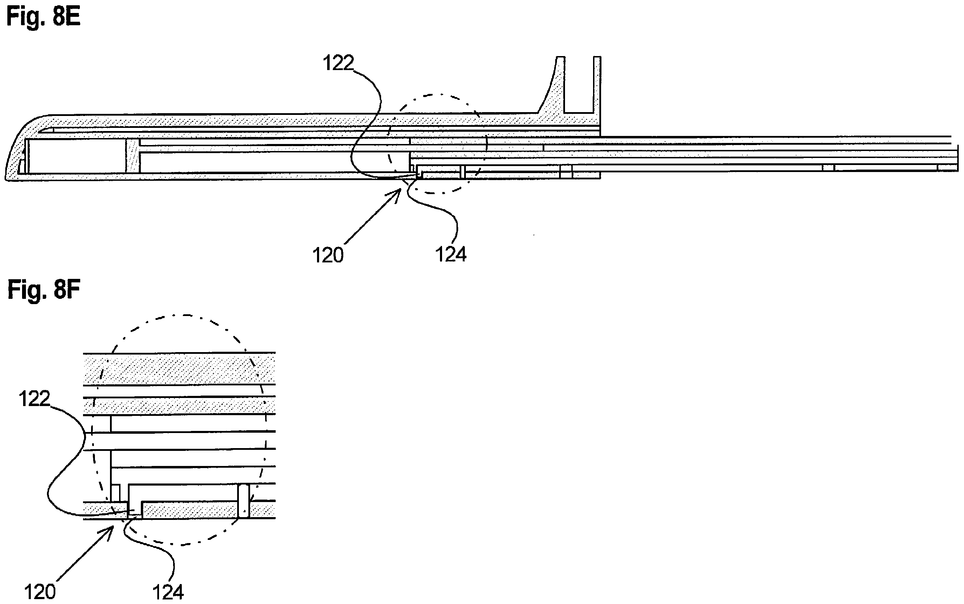

Die Figuren 8A und 8B zeigen eine erfindungsgemäße Ausführungsform der Injektionsvorrichtung 2, die einen Austausch der im Gehäuse 4 aufnehmbaren Spritze 16 und damit eine mehrfache Verwendung der Injektionsvorrichtung 2 ermöglicht. Hierzu ist eine lösbare Schließanordnung mit einem Schließelement 86 vorgesehen, das mit einer Schließaufnahme 88 in lösbaren Eingriff gebracht werden kann, um das erste äußere Gehäuseteil 6 mit dem zweiten äußeren Gehäuseteil 8 in der Schließstellung zu verbinden. Das Schließelement 86 weist hierbei einen elastisch gelagerten Schnapphaken 90 auf, der gemeinsam mit der Schließaufnahme 88 eine lösbare Schnappverbindung bildet. Alternativ hierzu wäre auch jede andere bekannte und geeignete lösbare Verbindung zwischen den beiden äußeren Gehäuseteilen 6, 8 verwendbar, wie beispielsweise eine reibschlüssige Verbindung. Zudem wäre auch eine lösbare Verbindung der äußeren Gehäuseteilen 6, 8 in der Schließstellung mittels der aufschiebbaren Fingeranlage 66 denkbar, die in diesem Falle derart mit dem Gehäuse 4 zusammenwirkt, dass sie nach einem durchgeführten Injektionsvorgang auch wieder von diesem abgenommen werden kann (nicht dargestellt).

Wie aus 8A und 8B ferner zu entnehmen ist, sind die Spritzenaufnahme 12 und die Stößelaufnahme 14 in dieser Ausführungsform der Injektionsvorrichtung 2 am ersten äußeren Gehäuseteil 6 gelagert, während das Betätigungselement 28 zusammen mit dem Zahnradgetriebe 32 am zweiten äußeren Gehäuseteil 8 gehalten ist. Hierbei bildet die Übertragungseinrichtung 30 zwischen der Stößelaufnahme 14 und dem Betätigungselement 28 eine Kopplungseinrichtung 92 aus, die in der dargestellten Offenstellung des Gehäuses 4 getrennt ist.

Die Kopplungseinrichtung 92 weist dabei ein stößelaufnahmeseitiges Kopplungsmittel 94 in Form einer Nut sowie ein betätigungselementseitiges Kopplungsgegenmittel 96 auf, das durch einen am Betätigungselement 28 gehaltenen Mitnehmer gebildet ist. In der dargestellten Offenstellung des Gehäuses 4 ist das stößelaufnahmeseitige Kopplungsmittel 94 vom betätigungselementseitigen Kopplungsgegenmittel 96 beabstandet angeordnet.

Durch Verschwenken der beiden äußeren Gehäuseteile 6, 8 in die Schließstellung kann das Kopplungsmittel 94 mit dem Kopplungsgegenmittel 96 in einen formschlüssigen Eingriff gebracht werden, sofern beide Elemente entsprechend auf gleicher Höhe angeordnet sind. Das Kopplungsgegenmittel 96 ist dabei teilweise um das Betätigungselement 28 herum anliegend ausgeformt, so dass dieses in der Schließstellung über das Kopplungsgegenmittel 96 an der Stößelaufnahme 14 abgestützt ist.

Um zu gewährleisten, dass das Gehäuse 4 nur in der exakt eingenommenen, dargestellten Ausgangsstellung der Stößelaufnahme 14 in die Schließstellung verbracht werden kann, sind am zweiten äußeren Gehäuseteil 8 Sicherungszapfen 98 vorgesehen. Diese können jeweils durch eine Durchtrittsöffnung 100 am ersten äußeren Gehäuseteil 6 hindurch mit einer ersten Eintrittsöffnung 102 zusammenwirken, die zusammen mit einer zweiten Eintrittsöffnung 104 entlang einer Sicherungsnut 106 in die Stößelaufnahme 14 eingelassen ist.

Die Sicherungszapfen 98 wirken dabei als Codierungsmittel, die nur bei exakter Anordnung der ersten Eintrittsöffnungen 102 auf Höhe der Durchtrittsöffnungen 100 in die jeweilige erste Eintrittsöffnung 102 eingeführt werden können, um das Gehäuse 4 zu schließen.

Ferner weisen die Sicherungszapfen 98 jeweils einen Hinterschnitt 108 auf, der derart dimensioniert ist, dass die Sicherungszapfen 98 die jeweilige Sicherungsnut 106 an Ihren Rändern hintergreifen und dabei entlang derselben in Richtung der zweiten Eintrittsöffnung 104 verlagert werden können. Hierdurch bilden die Sicherungszapfen 98 zusammen mit den Sicherungsnuten 106 zudem einen Sicherungsmechanismus, durch den ein Verbringen des Gehäuses 4 in die Offenstellung während einer Verlagerung des Betätigungselementes 28 verhindert werden kann.

Zusammen mit den zweiten Eintrittsöffnungen 104 fungieren die Sicherungszapfen 98 wiederum als Codierungsmittel, die ein Verbringen des Gehäuses 4 in die Offenstellung nach Durchführung eines Anwendungsvorganges nur dann erlauben, wenn die Eintrittsöffnungen 104 exakt auf Höhe der Durchtrittsöffnungen 100 angeordnet sind.

Um in der Offenstellung des Gehäuses 4 eine Verlagerung des Betätigungselementes 28 zu verhindern, sind zudem Festlegemittel 110 vorgesehen, mittels denen das Kopplungsgegenmittel 96 und folglich auch das Betätigungselement 28 in der Ausgangsstellung gegenüber dem Gehäuse 4 festgelegt sind, solange sich dieses in der dargestellten Offenstellung befindet.

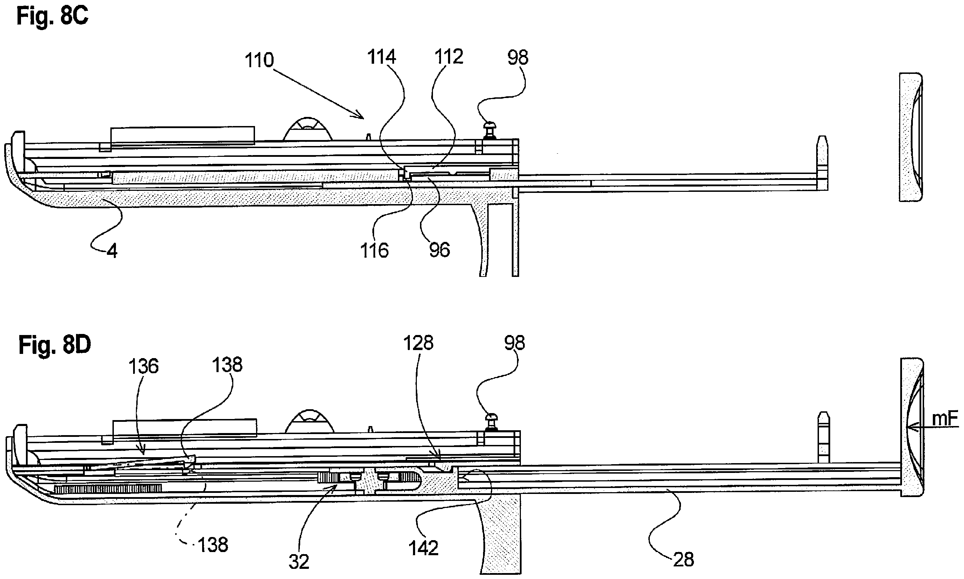

Wie insbesondere aus Figur 8C zu entnehmen ist, weisen diese Festlegemittel 110 jeweils eine gehäuseseitige Wippe 112 auf. Diese steht in der Offenstellung des Gehäuses 4 mit einem an einem ersten Ende ausgeformten Blockierhaken 114 in Eingriff mit einer Hakenaufnahme 116, die in das als Mitnehmer fungierende Kopplungsgegenmittel 96 eingelassen ist. Beim Verbringen des Gehäuses 4 in die Schließstellung werden die Wippen 112 jeweils an einem zweiten Ende von einem Entriegelungszapfen 118 beaufschlagt, der von dem jeweils anderen äußeren Gehäuseteil 6; 8 absteht, wie insbesondere aus Figur 8A zu entnehmen ist. Durch diese Beaufschlagung wird die jeweilige Wippe 112 derart verschwenkt, dass der Blockierhaken 114 außer Eingriff mit der jeweiligen Hakenaufnahme 116 kommt. Auf diese Weise wird die Blockierung des Betätigungselementes 28 über das Kopplungsgegenmittel 96 in der Schließstellung aufgehoben.

Um in der Offenstellung des Gehäuses 4 zudem auch eine Verlagerung der Spritzenaufnahme 12 und der Stößelaufnahme 14 zu verhindern, sind ferner zweite Festlegemittel 120 vorgesehen, die einen zweiten Blockierhaken 122 aufweisen, mittels dem die Stößelaufnahme 14 in der Offenstellung in Eingriff mit einer Gehäuseöffnung 124 steht. Hierdurch ist die Stößelaufnahme 14 und über den Verbindungsmechanismus 40 auch die Spritzenaufnahme 12 in der Offenstellung gegenüber dem Gehäuse 4 in ihrer Ausgangslage festgelegt.

Der Eingriff der Stößelaufnahme 14 an jeweils einem der äußeren Gehäuseteile 6; 8 kann beim Verbringen des Gehäuses 4 in die Schließstellung durch einen am jeweils anderen äußeren Gehäuseteil 8; 6 abstehenden zweiten Entriegelungszapfen 126 aufgehoben werden, mittels dem der zweite Blockierhaken 122 aus der Gehäuseöffnung 124 herausgedrückt werden kann (siehe Figur 8F).

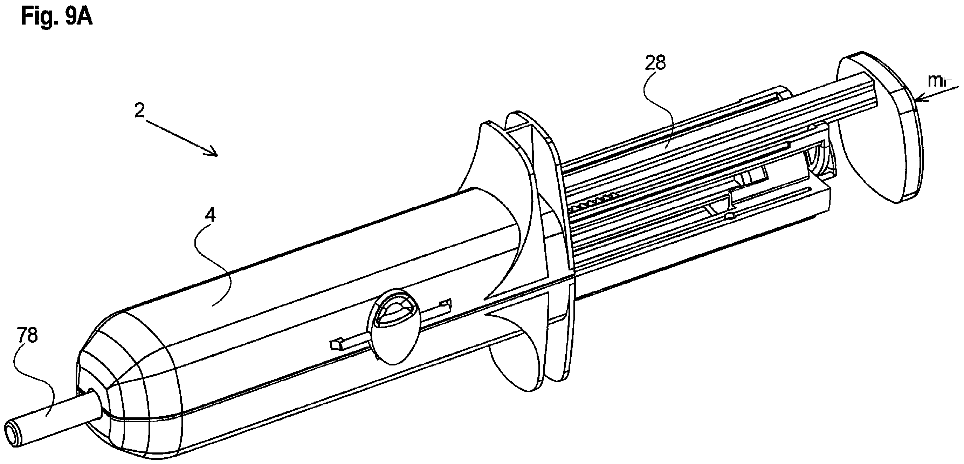

Nach Aufnahme der Spritze 16 und dem oben beschriebenen Verbringen des Gehäuses 4 in die Schließstellung befindet sich die Injektionsvorrichtung 2 in der betriebsbereiten Stellung gemäß Figur 9A. Nach Entfernen der Schutzkappe 78 und Anlage des stirnseitigen Endes der Injektionsvorrichtung 2 an dem betreffenden Patienten kann nun das Betätigungselement 28 mit der manuellen Kraft mF beaufschlagt werden, um den Injektionsvorgang entsprechend dem oben zum Ausführungsbeispiel nach den Figuren 1-7 beschriebenen Ablauf durchzuführen.

Wie aus Figur 8D zu entnehmen ist, muss dabei zu Beginn des Injektionsvorganges die manuelle Kraft mF ausreichend groß sein, um das Betätigungselement 28 außer Eingriff mit dem Startkrafterzeuger 128 bringen zu können, der durch eine Schnappverbindung zwischen dem Gehäuse 4 und dem Betätigungselement 28 gebildet ist. Sobald der durch den Startkrafterzeuger 128 definierte Widerstand durch die manuelle Kraft mF überwunden wird, läuft der Injektionsvorgang dann entsprechend dem oben beschriebenen Ablauf ab.

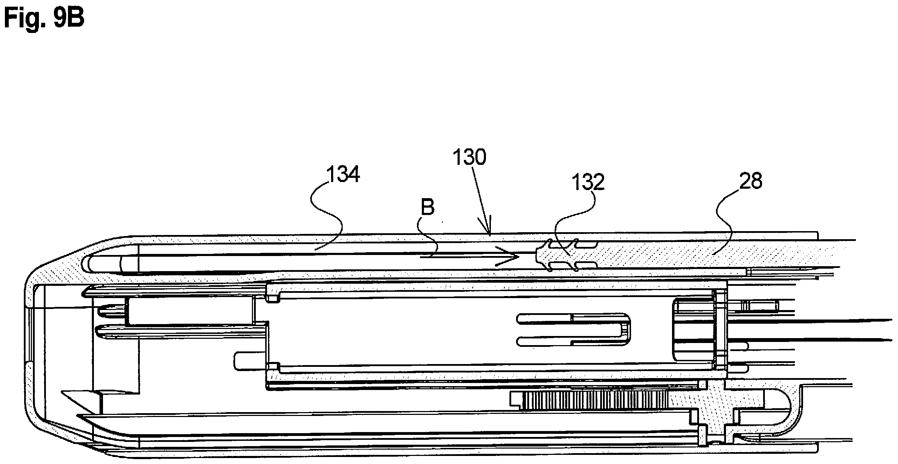

Wie aus Figur 9B zu entnehmen ist, weist die Injektionsvorrichtung 2 hierbei eine Dämpfungseinrichtung 130 auf, um die Vortriebsgeschwindigkeit des Betätigungselementes 28 während der Beaufschlagung mit der manuellen Kraft mF in einem vorbestimmten Bereich zu halten. Hierzu weist die Dämpfungseinrichtung 130 einen Verdrängungskörper 132 auf der mit dem Betätigungselement 28 bewegungsgekoppelt ist. Bei der Verlagerung des Betätigungselementes 28 wird dieser entlang eines Luftraumes 134 bewegt, wobei ein hierin enthaltener Luftkörper verdrängt werden muss. Über diese Luftverdrängung wird dabei eine vorbestimmte Bremskraft B erzeugt, die der manuellen Kraft mF entgegen gerichtet ist.

Nach vollständigem Durchlaufen des Injektionsvorganges wird das Betätigungselement 28 am Ende des Rückholhubes durch eine Endlagenverriegelung 136 fixiert, wodurch ein Aufziehen der Injektionsvorrichtung 2 in der Schließstellung verhindert wird. Wie insbesondere aus Figur 8A zu entnehmen ist, weist die Endlagenverriegelung 136 einen Hakenarm 138 auf, der beim Schließen des Gehäuses 4 durch zwei Spannzapfen 140 von einer Freigabestellung in eine Aktivstellung verbracht wird (siehe Fig. 8D). In dieser Aktivstellung verrastet der Hakenarm 138 mit einer Verriegelungsausnehmung 142 des Betätigungselementes 28 sobald sich dieses nach Abschluss des Rückholhubes in einer Endlage befindet.

Zum Lösen der Endlagenverriegelung 136 muss das Gehäuse 4 zunächst in die Offenstellung verbracht werden, wodurch sich der Hakenarm 138 durch elastische Rückstellkräfte zurück in die Freigabestellung begibt. Hiernach kann nun das Betätigungselement 28 wieder gegenüber dem Gehäuse 4 in seine Ausgangslage verlagert werden, um nach Austausch der Spritze 16 wieder einen neuen Injektionsvorgang durchführen zu können.

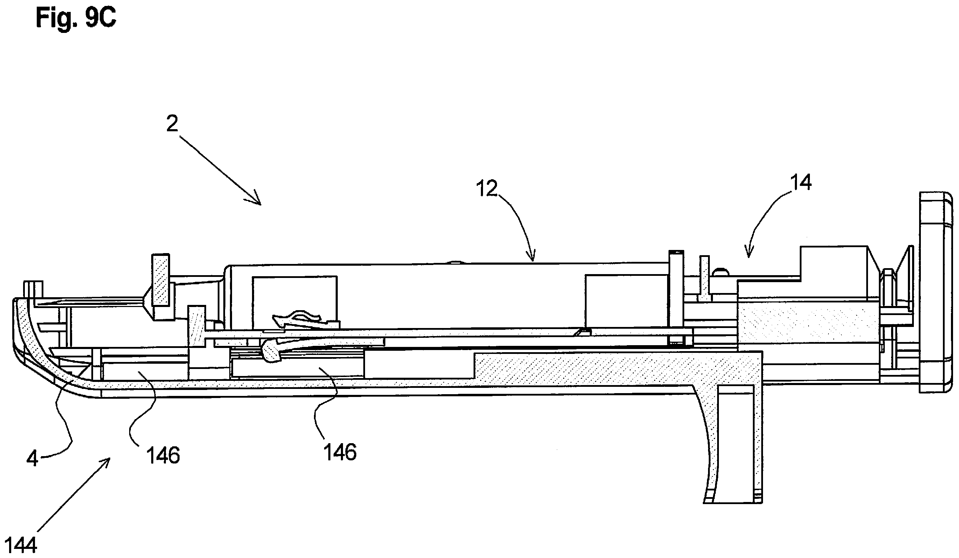

Wie aus Fig. 9C zu entnehmen ist, können die Spritzenaufnahme 12 und die Stößelaufnahme 14 zudem durch einen Verlagerungsmechanismus 144 in die Ausgangslage vorgespannt sein. Dieser Verlagerungsmechanismus 144 ist dabei in der dargestellten Ausführungsform der Injektionsvorrichtung 2 durch zwei Federeinrichtungen 146 gebildet, die zwischen dem Gehäuse 4 und der Spritzenaufnahme 12 bzw. der Stößelaufnahme 14 wirken. Durch die beim Öffnen des Gehäuses 4 aufgehobene Kopplung zwischen der Spritzenaufnahme 12 und der Stößelaufnahme 14 und die gleichzeitig erfolgende Trennung der Kopplungseinrichtung 92 können die Federeinrichtungen 146 die Spritzenaufnahme 12 und die Stößelaufnahme 14 selbsttätig in ihre jeweilige Ausgangsposition drücken.

In dieser nun eingenommenen Ausgangslage kann dann eine neue Spritze 16 eingesetzt werden, um den nächsten Injektionsvorgang durchführen zu können.

Um hierbei auch Spritzen 16 von unterschiedlicher Länge verwenden zu können, kann die Verwendung von Adapterstücken vorgesehen werden, die in die Spritzenaufnahme 12 und/oder in die Stößelaufnahme 14 eingesetzt werden (nicht dargestellt). Alternativ hierzu ist es zudem auch möglich, die Spritzenaufnahme 12 und/oder Stößelaufnahme 14 austauschbar auszubilden, sodass diese mit unterschiedlichen Größen in die Injektionsvorrichtung 2 eingesetzt werden können (nicht dargestellt).

Diese für die mehrfach verwendbare Injektionsvorrichtung 2 dargestellten Maßnahmen zur Anpassung an unterschiedliche Spritzengrößen können dabei, wie auch die oben beschriebenen Sicherungsmaßnahmen zu Vermeidung einer Fehlbedienung, ebenfalls an der als Einwegprodukt ausgelegten Injektionsvorrichtung 2 gemäß der Figuren 1 bis 7 umgesetzt werden.

## Patentansprüche

1. Injektionsvorrichtung (2)
mit einem Gehäuse (4), in dem eine Spritze (16) aufnehmbar ist, die eine Injektionsnadel (18), eine Aufnahmekammer (20), einen Kolben (22) und einen Spritzenstößel (24) aufweist,
und einer Steuerungsanordnung (26) zur gesteuerten Betätigung der Spritze (16) während eines Anwendungsablaufes, der wenigstens einen Einstechhub, einen Injektionshub und einen Rückholhub umfasst,
wobei die Steuerungsanordnung (26) ein Betätigungselement (28) zur Beaufschlagung durch eine manuelle Antriebskraft (mF),
eine gegenüber dem Gehäuse (4) verschiebbare Spritzenaufnahme (12), an der die Aufnahmekammer (20) festlegbar ist,
eine gegenüber dem Gehäuse (4) verschiebbare Stößelaufnahme (14), an der der Spritzenstößel (24) festlegbar ist,
sowie eine Übertragungseinrichtung (30) aufweist, die ein Zahnradgetriebe (32) zur Koppelung der Stößelaufnahme (14) mit dem Betätigungselement (28) aufweist, mittels dem in Abhängigkeit von einer Relativbewegung des Betätigungselementes (28) gegenüber dem Gehäuse (4) die Stößelaufnahme (14) antreibbar ist,
**dadurch gekennzeichnet, dass** das Gehäuse (4) ein erstes äußeres Gehäuseteil (6) und ein zweites äußeres Gehäuseteil (8) aufweist, die über ein zwischen ihnen vorgesehenes Schwenklager (10) aneinander zwischen einer Offenstellung, in der die Spritzenaufnahme (12) zugänglich ist, und einer Schließstellung, in der die Spritzenaufnahme (12) verschlossen ist, verlagerbar gehalten sind wobei die Übertragungseinrichtung (30) zwischen der Stößelaufnahme (14) und dem Betätigungselement (28) eine in der Offenstellung getrennte Kopplungseinrichtung (92) mit einem stößelaufnahmeseitigen Kopplungsmittel (94) und einem betätigungselementseitigen Kopplungsgegenmittel (96) aufweist, die in der Schließstellung in einem in und entgegen der Injektionsrichtung (R) wirkenden formschlüssigen Eingriff bringbar sind.

2. Injektionsvorrichtung nach Anspruch 1, **dadurch gekennzeichnet, dass** die beiden äußeren Gehäuseteile (6, 8) zueinander sowohl in die Offenstellung als auch in die Schließstellung vorspannbar sind.

3. Injektionsvorrichtung nach Anspruch 1 oder 2, **dadurch gekennzeichnet, dass** das zweite äußere Gehäuseteil (8) in der Schließstellung mit dem ersten äußeren Gehäuseteil (6) dauerhaft oder lösbar verbindbar ist.

4. Injektionsvorrichtung nach einem der Ansprüche 1 bis 3, **dadurch gekennzeichnet, dass** zwischen dem ersten äußeren Gehäuseteil (6) und dem zweiten äußeren Gehäuseteil (8) ein Endanschlag (56) ausgebildet ist, über den die beiden Gehäuseteile (6, 8) in der Offenstellung aneinander anlegbar sind.

5. Injektionsvorrichtung nach einem der Ansprüche 1 bis 4, **dadurch gekennzeichnet, dass** das erste äußere Gehäuseteil (6) und das zweite äußere Gehäuseteil (8) beim Verschwenken von der Offenstellung in die Schließstellung aneinander geführt sind.

6. Injektionsvorrichtung nach einem der Ansprüche 1 bis 5, **dadurch gekennzeichnet, dass** das Schwenklager (10) eine Sollbruchstelle ausbildet.

7. Injektionsvorrichtung nach einem der Ansprüche 1 bis 6, **dadurch gekennzeichnet, dass** eine Betätigungssicherung (70) vorgesehen ist, die eine Verlagerung des Betätigungselementes (28) in der Offenstellung blockiert.

8. Injektionsvorrichtung nach einem der Ansprüche 1 bis 7, **dadurch gekennzeichnet, dass** die Spritzenaufnahme (12) und die Stößelaufnahme (14) am ersten äußeren Gehäuseteil (6) und das Betätigungselement (28) und das Zahnradgetriebe (32) am zweiten äußeren Gehäuseteil (8) gehalten sind.

9. Injektionsvorrichtung nach einem der Ansprüche 1 bis 8, **dadurch gekennzeichnet, dass** Adaptermittel vorgesehen sind, mittels denen die Spritzenaufnahme (12) und/oder die Stößelaufnahme (14) an unterschiedliche Spritzen (16) anpassbar ist.

10. Injektionsvorrichtung nach einem der Ansprüche 1 bis 9, **dadurch gekennzeichnet, dass** zwischen den äußeren Gehäuseteilen (6, 8) und der Spritzenaufnahme (12) und/oder der Stößelaufnahme (14) Codierungsmittel vorgesehen sind, die eine Ausgangsstellung definieren, in der die Gehäuseteile (6, 8) in die Schließstellung verbringbar sind.

11. Injektionsvorrichtung nach einem der Ansprüche 1 bis 10, **dadurch gekennzeichnet, dass** zwischen dem ersten äußeren Gehäuseteil (6) und dem zweiten äußeren Gehäuseteil (8) ein Sicherungsmechanismus zur Verhinderung eines Öffnens der beiden Gehäuseteile (6, 8) während des Betriebs der Injektionsvorrichtung (2) vorgesehen ist.

12. Injektionsvorrichtung nach einem der Ansprüche 1 bis 11, **dadurch gekennzeichnet, dass** zwischen der Spritzenaufnahme (12) und der Stößelaufnahme (14) ein lösbarer Verbindungsmechanismus (40) angeordnet ist, der ein Einrastelement aufweist, das in einer Einstechstellung und in einer Rückholstellung mit einer Einrastaufnahme (46, 82) in Eingriff bringbar ist.

13. Injektionsvorrichtung nach einem der Ansprüche 10 bis 12, **dadurch gekennzeichnet, dass** ein Verlagerungsmechanismus (144) vorgesehen ist, mittels dem die Stößelaufnahme (14) bei Verlagerung der äußeren Gehäuseteile (6, 8) in die Offenstellung selbsttätig in die Ausgangsstellung verbringbar ist.

14. Injektionsvorrichtung nach einem der Ansprüche 1 bis 13, **dadurch gekennzeichnet, dass** das Betätigungselement (28) mit einer Dämpfungseinrichtung (130) gekoppelt ist.

15. Injektionsvorrichtung nach einem der Ansprüche 1 bis 14, **dadurch gekennzeichnet, dass** eine Endlagenverriegelung (136) zur Festlegung des Betätigungselementes (28) in einer bei Abschluss eines Injektionsvorganges eingenommenen Endlage vorgesehen ist und die Endlagenverriegelung (136) durch Verlagerung der äußeren Gehäuseteile (6, 8) in die Offenstellung in eine Freigabestellung verbringbar ist.

## Claims

1. Injection device (2)
with a housing (4), in which a syringe (16) can be held, which has an injection needle (18), a holding chamber (20), a plunger tip (22), and a syringe plunger (24),
and a control assembly (26) for the controlled actuation of the syringe (16) during an application process, which comprises at least one insertion stroke, an injection stroke, and a return stroke,
wherein the control assembly (26) has an actuation element (28) for receiving a manual driving force (mF),
a syringe holder (12) which can be slid in relation to the housing (4) and to which the holding chamber (20) can be fastened,
a plunger holder (14) which can be slid in relation to the housing (4) and to which the syringe plunger (24) can be fastened,
as well as a transmission device (30), which has a gearing (32) for coupling the plunger holder (14) to the actuation element (28), by means of which gearing the plunger holder (14) can be driven in accordance with a motion of the actuation element (28) relative to the housing (4),
**characterised in that** the housing (4) comprises a first outer housing part (6) and a second outer housing part (8), which, by means of a pivot bearing (10) provided between them, can be held such as to be movable between an open position, in which the syringe holder (12) is accessible, and a closed position, in which the syringe holder (12) is enclosed, wherein the transmission device (30) comprises a coupling device (92) between the plunger holder (14) and the actuation element (28), which is separated in the open position, with a coupling means (94) on the side with the plunger holder, and with coupling counter-means (96) on the actuation element side, which in the closed position can be brought into positive-fit engagement taking effect in and against the injection direction (R).

2. Injection device according to claim 1, **characterised in that** the two outer housing parts (6,8) can be tensioned against one another both in the open position as well as in the closed position.

3. Injection device according to claim 1 or 2, **characterised in that,** in the closed position, the second outer housing part (8) can be connected permanently or detachably to the first outer housing part (6).

4. Injection device according to any one of claims 1 to 3, **characterised in that** an end stop (56) is formed between the first outer housing part (6) and the second outer housing part (8), by means of which the two housing parts (6, 8) can be laid on one another in the open position.

5. Injection device according to any one of claims 1 to 4, **characterised in that** the first outer housing part (6) and the second outer housing part (8) are guided to one another when pivoted out of the open position into the closed position.

6. Injection device according to any one of claims 1 to 5, **characterised in that** the pivot bearing (10) forms a predetermined break point.

7. Injection device according to any one of claims 1 to 6, **characterised in that** an actuation securing element (70) is provided, which in the open position blocks a repositioning of the actuation element (28).

8. Injection device according to any one of claims 1 to 7, **characterised in that** the syringe holder (12) and the plunger holder (14) are held at the first outer housing part (6) and the actuation element (28) and the gearing (32) are held at the second outer housing part (8).

9. Injection device according to any one of claims 1 to 8, **characterised in that** adapter means are provided, by means of which the syringe holder (12) and/or the plunger holder (14) can be adapted to different syringes (16).

10. Injection device according to any one of claims 1 to 9, **characterised in that** coding means are provided between the outer housing parts (6, 8) and the syringe holder (12) and/or the plunger holder (14), which define a starting position, in which the housing parts (6, 8) can be brought into the closed position.

11. Injection device according to any one of claims 1 to 10, **characterised in that** a securing mechanism is provided between the first outer housing part (6) and the second outer housing part (8), in order to prevent an opening of the two housing parts (6, 8) during the operation of the injection device (2).

12. Injection device according to any one of claims 1 to 11, **characterised in that** a detachable connection mechanism (40) is arranged between the syringe holder (12) and the plunger holder (14), which comprises a latch element which in an insertion position and in a return position can be brought into engagement with a first latch receiver (46, 82).

13. Injection device according to any one of claims 10 to 12, **characterised in that** a repositioning mechanism (144) is provided, by means of which, at the repositioning of the outer housing parts (6, 8) into the open position, the plunger holder (14) can be automatically be brought into the starting position.

14. Injection device according to any one of claims 1 to 13, **characterised in that** the actuation element (28) is coupled to a damping device (130).

15. Injection device according to any one of claims 1 to 14, **characterised in that** an end position locking element (136) is provided, for securing the actuation element (28) in an end position adopted at the concluding of an injection procedure, and the end position locking element (136) can be brought in a release position by the repositioning of the outer housing parts (6, 8) into the open position.

## Revendications

1. Dispositif d'injection (2)
comprenant un boîtier (4), dans lequel peut être logée une seringue (16) qui comprend une aiguille d'injection (18), une chambre de réception (20), un piston (22) et un poussoir de seringue (24),
et un ensemble de commande (26) pour l'actionnement commandé de la seringue (16) au cours d'un processus d'utilisation comprenant au moins une course de piqûre, une course d'injection et une course de retrait,
l'ensemble de commande (26) comprenant un élément d'actionnement (28) destiné à être soumis à une force d'entraînement manuelle (mF), un logement de seringue (12) qui est déplaçable par rapport au boîtier (4) et auquel la chambre de réception (20) peut être fixée, un logement de poussoir (14) qui est déplaçable par rapport au boîtier (4) et auquel le poussoir d'injection (24) peut être fixé, ainsi qu'un dispositif de transmission (30), qui comprend une transmission à roue dentée (32) pour le couplage du logement de poussoir (14) avec l'élément d'actionnement (28), au moyen de laquelle le logement de poussoir (14) peut être entraîné en fonction d'un mouvement relatif de l'élément d'actionnement (28) par rapport au boîtier (4),
**caractérisé en ce que** le boîtier (4) comprend une première partie de boîtier extérieure (6) et une seconde partie de boîtier extérieure (8), qui sont maintenues ensemble de façon déplaçable par l'intermédiaire d'un support pivotant (10) prévu entre celles-ci entre une position ouverte, dans laquelle le logement de seringue (12) est accessible, et une position fermée, dans laquelle le logement de seringue (12) est fermée, le dispositif de transmission (30) comprenant, entre le logement de poussoir (14) et l'élément d'actionnement (28), un dispositif de couplage (92), séparé dans la position ouverte, muni d'un moyen de couplage (94) côté logement de poussoir et d'un moyen de couplage (96) côté élément d'actionnement, qui peuvent être amenés dans un engagement par complémentarité de forme agissant dans et à l'encontre de la direction d'injection (R) dans la position fermée.

2. Dispositif d'injection selon la revendication 1, **caractérisé en ce que** les deux parties de boîtier extérieures (6, 8) peuvent être précontraintes l'une par rapport à l'autre aussi bien dans la position ouverte que la position fermée.

3. Dispositif d'injection selon la revendication 1 ou 2, **caractérisé en ce que** la deuxième partie de boîtier extérieure (8) peut être reliée de manière permanente ou amovible avec la première partie de boîtier extérieure (6) dans la position fermée.

4. Dispositif d'injection selon l'une des revendications 1 à 3, **caractérisé en ce que,** entre la première partie de boîtier extérieure (6) et la deuxième partie de boîtier extérieure (8), il est formé une butée de fin de course (56), par l'intermédiaire de laquelle les deux parties de boîtier (6, 8) peuvent être apposées l'une contre l'autre dans la position ouverte.

5. Dispositif d'injection selon l'une des revendications 1 à 4, **caractérisé en ce que** la première partie de boîtier extérieure (6) et la deuxième partie de boîtier extérieure (8) sont guidées l'une contre l'autre lors du pivotement de la position ouverte à la position fermée.

6. Dispositif d'injection selon l'une des revendications 1 à 5, **caractérisé en ce que** le support pivotant (10) forme une zone de faiblesse.

7. Dispositif d'injection selon l'une des revendications 1 à 6, **caractérisé en ce qu'il** est prévu une sécurité d'actionnement (70) qui bloque un déplacement de l'élément d'actionnement (28) dans la position ouverte.

8. Dispositif d'injection selon l'une des revendications 1 à 7, **caractérisé en ce que** le logement de seringue (12) et le logement de poussoir (14) sont maintenus sur la première partie de boîtier extérieure (6) et l'élément d'actionnement (28) et la transmission par roue dentée (32) sont maintenus sur la deuxième partie de boîtier extérieure (8).

9. Dispositif d'injection selon l'une des revendications 1 à 8, **caractérisé en ce qu'il** est prévu des moyens adaptateurs, au moyens desquels le logement de seringue (12) et/ou le logement de poussoir (14) peut être adapté à différentes seringues (16).

10. Dispositif d'injection selon l'une des revendications 1 à 9, **caractérisé en ce que,** entre les parties de boîtier extérieures (6, 8) et le logement de seringue (12) et/ou le logement de poussoir (14), il est prévu des moyens de codage qui définissent une position de sortie, dans laquelle les parties de boîtier (6, 8) peuvent être amenées dans la position fermée.

11. Dispositif d'injection selon l'une des revendications 1 à 10, **caractérisé en ce que,** entre la première partie de boîtier extérieure (6) et la deuxième partie de boîtier extérieure (8), il est prévu un mécanisme de sécurité pour empêcher une ouverture des deux parties de boîtier (6, 8) au cours du fonctionnement du dispositif d'injection (2).

12. Dispositif d'injection selon l'une des revendications 1 à 11, **caractérisé en ce que,** entre le logement de seringue (12) et le logement de poussoir (14), il est agencé un mécanisme de liaison amovible (40) comprenant un élément d'enclenchement, qui peut être amené en engagement avec un logement d'enclenchement (46, 82) dans une position de piqûre et dans une position de retrait.

13. Dispositif d'injection selon l'une des revendications 10 à 12, **caractérisé en ce qu'il** est prévu un mécanisme de déplacement (144), au moyen duquel le logement de poussoir (14) peut être amené dans la position de sortie automatiquement lors du déplacement des parties de boîtier extérieures (6, 8) dans la position ouverte.

14. Dispositif d'injection selon l'une des revendications 1 à 13, **caractérisé en ce que** l'élément d'actionnement (28) est couplé avec un dispositif d'amortissement (130).

15. Dispositif d'injection selon l'une des revendications 1 à 14, **caractérisé en ce qu'il** est prévu un verrouillage de fin de course (136) pour l'arrêt de l'élément d'actionnement (28) dans une position de fin de course déterminée par l'issue d'un processus d'injection, et le verrouillage de fin de course (136) peut être amené dans une position de libération par le déplacement des parties de boîtier extérieures (6, 8) dans la position ouverte.
